# EUROPEAN PATENT APPLICATION

(11) **EP 4 512 891 A1**
(43) Date of publication of application: **26.02.2025**
(21) Application number: 22938043.1
(22) Date of filing: 17.05.2022
(51) Int. Cl.: C12N 9/10, C12N 15/54, C12N 15/70, C12N 15/81, C12N 1/19, C12N 1/21, C12P 13/00, C12R 1/19, C12R 1/645

(54) **TRANSAMINASE MUTANT AND USE THEREOF**

(30) Priority: 21.04.2022 CN 202210426193
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); ZHANG, Kejian, Tianjin 300457 (CN); WANG, Lei, Tianjin 300457 (CN); ZHAO, Junqi, Tianjin 300457 (CN); LING, Jun, Tianjin 300457 (CN); XU, Xinsheng, Tianjin 300457 (CN); LI, Rui, Tianjin 300457 (CN); WANG, Zujian, Tianjin 300457 (CN)
(74) Representative: Delbarba, Andrea
(86) International application number: PCT/CN2022/093313
(87) International publication number: WO 2023/201809

(57) **Abstract**

Provided are a transaminase mutant and use thereof. The transaminase mutant is obtained by mutation occurred in an amino acid sequence as shown in SEQ ID NO:1, and the mutation includes at least T291A; or an amino acid sequence of the transaminase mutant has the mutation in the mutated amino acid sequence, and has more than 80% of identity with the mutated amino acid sequence, preferably has 85% of identity, and more preferably has 90% of identity. The transaminase mutant expands a substrate spectrum of enzymes, improves stereoselectivity, and improves the catalytic capacity of a large steric hindrance substrate; and at the same time, the stability of the enzymes is improved, and a catalytic reaction may be performed to produce chiral amines at high solvent concentrations and high temperatures.

## Description

### Technical Field

The present disclosure relates to the field of biotechnologies, and in particular, to a transaminase mutant and use thereof.

### Background

Chiral amines are a class of compounds containing amino groups in chiral centers of small molecule compounds, are key intermediates of many pharmaceuticals and pesticides, and are receiving more and more attention in the production of pharmaceuticals and industrial and agricultural production. The chiral amines are important intermediates in synthesis of neurologic drugs, cardiovascular drugs, antihypertensive drugs, anti-infective drugs, and vaccines, etc. At present, 40% to 50% of chiral drugs are chiral amine compounds, and the efficient and convenient synthesis of the chiral amine compounds is an important direction of research on organic synthetic chemistry. For example, a diabetes drug sitagliptin (Merck and Codexis) and a broad-spectrum contact herbicide glufosinate-ammonium (Bayer) both have chiral amine chemistry modules.

At present, methods for preparing the chiral amines mainly include chemical methods, biological separation methods, and biological asymmetric synthesis methods. Using the chemical method to synthesize the chiral amines often requires the use of expensive metal catalysts and special solvents, with high production costs, low enantioselectivity, and some environmental pollution; and using an enzymatic method to prepare chiral aromatic amines has the characteristics of being high in catalytic efficiency, strong in stereoselectivity, mild in reaction condition, friendly to environment, etc., which is in line with the general trend of green synthesis, and thus has become a hot spot of current research. Enzymes currently used for the preparation of the chiral aromatic amines are mainly lipases and transaminases. Compared to the fact that a maximum theoretical yield for preparation of high-purity chiral amines by a chiral separation method using a biological catalyst (lipases) is only 50%, a theoretical yield of the biological asymmetric synthesis method (transaminases) reaches up to 100%, and thus has strong application prospects in the field of production industries, and related research has received extensive attention. With the advantages of high enantioselectivity and regioselectivity, broad substrate spectra, and no need for additional expensive coenzymes, ω-transaminases (ω-TAs) have become one of the most important industrial enzymes used in industry for the production of the chiral amines.

The transaminases, also referred to as aminotransferases, belong to transferases that are capable of reversibly catalyzing an aminotransfer reaction between ketone groups and amino groups. When a substrate or a product contains α amino acids in a transamination reaction catalyzed by such enzymes, the enzymes are referred to as α transaminases, otherwise referred to as ω transaminases. Products of the α-transaminases are generally only α amino acids, while the ω-transaminases are able to aminate keto acids, aldehydes, and ketones and have the characteristics of being high in stereoselectivity and renewable in cofactor, such that the ω-transaminases are more widely applied to synthesize pharmaceutical and pesticide intermediates. However, few of the currently reported wild ω-TAs can be directly used in the pharmaceutical industry, which is mainly limited by the instability of the enzymes and unfavorable reaction equilibrium.

### Summary

The present disclosure is intended to provide a transaminase mutant and use thereof, so as to improve activity of transaminase.

In order to realize the above purpose, an aspect of the present disclosure provides a transaminase mutant. The transaminase mutant is obtained by mutation occurred in an amino acid sequence as shown in SEQ ID NO:1, and the mutation includes at least T291A; or the amino acid sequence of the transaminase mutant has a mutation site in the mutated amino acid sequence, and has more than 80% of identity with the mutated amino acid sequence, preferably has 85% of identity, more preferably has 90% of identity, further preferably has 95% of identity, and further preferably has 99% of identity.

Further the mutation includes T291A and at least one of the following mutation sites: V17S, A56S, A56P, F64L, Y69L, Y69M, Y69C, Y69G, T70A, T70G, T70M, T70C, T70S, Y75F, G77E, G77S, G77V, G77T, G77L, I78H, T106S, M130I, M130L, N132W, N132A, T134I, T134A, T134V, Y139S, G140C, S141G, K142G, K142V, Y144I, Y144V, Y144P, Y144L, Y144M, Y144F, E145N, E145S, E145L, E145H, E145D, A148T, A148Q, H151A, H151D, H151Y, H151Q, R152K, R152T, Y158I, Y158V, I160G, I160T, I160S, I160V, I160C, I160T, Q163S, Q163T, Q163N, Q163A, W164F, I165L, I165H, I165A, I165V, P167A, P167K, Q171T, W200A, S204L, S204M, R216E, R216K, R216N, G232F, F233Y, A242S, A242Q, V244G, V244R, V244Y, V244S, V244K, V244M, T270G, T270H, S278L, S278R, S278K, E282C, E282R, E282K, T291A, T291Q, T291C, T291S, T291M, T291T, G292A, G292S, or M326Q.

Further, the mutation further includes at least one of the following mutation site combinations: T291A+S290L, T291A+S290V, T291A+S290T, T291A+G292A, T291A+G292L, T291A+G292I, T291A+G292D, T291A+G292A+Y75T, T291A+G292A+G77E, T291A+G292A+M130I, T291A+G292A+M130L, T291A+G292A+Y144S, T291A+G292A+Y144G, T291A+G292A+Y144I, T291A+G292A+Y144V, T291A+G292A+Y144P, T291A+G292A+Y144L, T291A+G292A+Y144H, T291A+G292A+Q163S, T291A+G292A+Q163T, T291A+G292A+Q163L, T291A+G292A+Q163N, T291A+G292A+Q163V, T291A+G292A+W164F, T291A+G292A+W164R, T291A+G292A+W164L, T291A+G292A+W164T, T291A+G292A+I165A, T291A+G292A+I165V, T291A+G292A+I165L, T291A+G292A+W200G, T291A+G292A+T70C, T291A+G292A+T70C+W164F, T291A+G292A+T70C+F233Y, T291A+G292A+T70C+I165V, T291A+G292A+T70C+Y144M+G145N, T291A+G292A+T70C+Y144M+G145S, T291A+G292A+T70C+G145L, T291A+G292A+T70C+Y69L, T291A+G292A+T70C+T142G, T291A+G292A+T70C+I160G, T291A+G292A+T70C+I160T, T291A+G292A+T70C+I160S, T291A+G292A+T70C+I160V, T291A+G292A+T70C+I160C, T291A+G292A+T70C+S204L, T291A+G292A+T70C+S204M, T291A+G292A+T70C+V244G, T291A+G292A+T70C+V244R, T291A+G292A+T70C+V244Y, T291A+G292A+T70C+V244S, T291A+G292A+T70C+V244K, T291A+G292A+T70C+V244M, T291A+G292A+T70C+I160G+H151A, T291A+G292A+T70C+I160G+H151D, T291A+G292A+T70C+I160G+H151Y, T291A+G292A+T70C+I160G+H151Q, T291A+G292A+T70C+I160G, T291A+G292A+T70C+I160G+S204L, T291A+G292A+T70C+I160G+V244S, T291A+G292A+T70C+I160G+S204L+V244S, T291A+G292A+T70C+I160G+T134I, T291A+G292A+T70C+I160G+A242S, T291A+G292A+T70C+I160G+A242Q, T291A+G292A+T70C+I160G+T270G, T291A+G292A+T70C+I160G+T270H, T291A+G292A+T70C+I160G+S278L, T291A+G292A+T70C+I160G+S278R, T291A+G292A+T70C+I160G+S278K, T291A+G292A+T70C+I160G+E282C, T291A+G292A+T70C+I160G+E282R, T291A+G292A+T70C+I160G+E282K, T291A+G292A+T70C+I160G+M326Q, T291A+G292A+T70C+I160G+V17S, T291A+G292A+T70C+I160G+A56S, T291A+G292A+T70C+I160G+F64L, T291A+G292A+T70C+I160G+P167A, T291A+G292A+T70C+I160G+P167K, T291A+G292A+T70C+I160G+R216E, T291A+G292A+T70C+I160G+R216K, T291A+G292A+T70C+I160G+S204L+V244S+T134I, T291A+G292A+T70C+I160G+S204L+V244S+M326Q, T291A+G292A+T70C+I160G+S204L+V244S+T134I, T291A+G292A+T70C+I160G+S204L+V244S+T134I+M326Q, T291A+G292A+T70C+I160G+S204L+V244S+T134I+M326Q, T291A+G292A+T70C+I160G+S204L+V244S+T134I+M326Q+Y158I, T291A+G292A+T70C+I160G+S204L+V244S+T134I+M326Q+Y158V, T291A+G292A+T70C+I160G+S204L+V244S+T134I+M326Q+R216N, T291A+G292A+T70C+I160G+S204L+V244S+T134I+M326Q+R216K, T291A+G292A+L232F, T291A+G292A+F233W, T291A+G292A+F233Y, T291A+G292A+T70G, T291A+G292A+T70M, T291A+G292A+T70A, T291A+G292A+T70S, T291A+G292A+T70C, T291A+G292A+W164Y, T291A+G292A+T70C+I160G+S204L+V244S+T134I+M326Q+G77V, T291A+G292A+T70C+I160G+S204L+V244S+T134I+M326Q+W200A, T291A+G292A+T70C+I160G+S204L+V244S+T134I+M326Q+Y144L, T291A+G292A+T70C+I160G+S204L+V244S+T134I+M326Q+Y144L+G77T, T291A+G292A+T70C+I160G+S204L+V244S+T134I+M326Q+Y144L+G77L, T291A+G292A+T70C+I160G+S204L+V244S+T134I+M326Q+A292G, T291A+G292A+T70C+I160G+S204L+V244S+T134I+M326Q+A292G+G77T, T291A+G292A+T70C+I160G+S204L+V244S+T134I+M326Q+W200A+A56P, T291A+G292A+T70C+I160G+S204L+V244S+T134I+M326Q+W200A+A56P+N132A, T291A+G292A+T70C+I160G+S204L+V244S+T134I+M326Q+W200A+A56P+G77S, T291A+G292A+T70C+I160G+S204L+V244S+T134I+M326Q+W200A+A56P+G77T, T291A+G292A+T70C+I160G+S204L+V244S+T134I+M326Q+W200A+A56P+Y144F, T291A+G292A+T70C+I160G+S204L+V244S+T134I+M326Q+Y144L+G77T, T291A+G292A+T70C+I160G+S204L+V244S+T134I+M326Q+Y144L+G77T-12aa, T291A+G292A+T70C+I160G+S204L+V244S+T134I+M326Q+A292G+G77T-12aa, T291A+G292A+T70C+I160G+S204L+V244S+T134I+M326Q+Y144L+G77T+L144W-12aa, T291A+G292A+T70C+I160G+S204L+V244S+T134I+M326Q+Y144L+G77T+L144P-12aa, T291A+G292A+T70C+I160G+S204L+V244S+T134I+M326Q+Y144L+G77T+N132R-12aa, T291A+G292A+T70C+I160G+S204L+V244S+T134I+M326Q+Y144L+G77T+I165L-12aa, T291A+G292A+T70C+I160G+S204L+V244S+T134I+M326Q+Y144L+G77T+I165H-12aa, T291A+G292A+T70C+I160G+S204L+V244S+T134I+M326Q+Y144W+G77T+I165L-12aa, T291A+G292A+T70C+I160G+S204L+V244S+T134I+M326Q+Y144W+G77T+I165H-12aa, T291A+G292A+T70C+I160G+S204L+V244S+T134I+M326Q+Y144W+G77T+I165L-12aa, T291A+G292A+T70C+I160G+S204L+V244S+T134I+M326Q+Y144W+G77T+I165L+I78V-12aa, T291A+G292A+T70C+I160G+S204L+V244S+T134I+M326Q+Y144W+G77T+I165L+A291I-12aa, T291A+G292A+T70C+I160G+S204L+V244S+T134I+M326Q+Y144W+G77T+I165L+A292S-12aa, T291A+G292A+T70C+I160G+S204L+V244S+T134I+M326Q+Y144W+G77T+I165L+A292S+I78H-12aa, T291A+G292A+T70C+I160G+S204L+V244S+T134I+M326Q+Y144W+G77T+I165L+A292S+S278 R-12aa, T291A+G292A+T70C+I160G+S204L+V244S+T134I+M326Q+Y144W+G77T+I165L+A292S+S278 R-12aa, T291A+G292A+T70C+I160G+S204L+V244S+T134I+M326Q+Y144W+G77T+I165L+A292S+S278 R+T142V-12aa, T291A+G292A+T70C+I160G+S204L+V244S+T134I+M326Q+Y144W+G77T+I165L+A2925+5278 R+G 145H-12aa, T291A+G292A+T70C+I160G+S204L+V244S+T134I+M326Q+Y144W+G77T+I165L+A2925+5278 R+G1450-12aa, T291A+G292A+T70C+I160G+S204L+V244S+T134I+M326Q+Y144W+G77T+I165L+A292S+S278 R+A148T-12aa, T291A+G292A+T70C+I160G+S204L+V244S+T134I+M326Q+Y144W+G77T+I165L+A292S+S278 R+A 148Q-12aa, T291A+G292A+T70C+I160G+S204L+V244S+T134I+M326Q+Y144W+G77T+I165L+A292S+S278 R+R152K-12aa, T291A+G292A+T70C+I160G+S204L+V244S+T134I+M326Q+Y144W+G77T+I165L+A2925+5278 R+R152T-12aa, T291A+G292A+T70C+I160G+S204L+V244S+T134I+M326Q+Y144W+G77T+I165L+A2925+5278 R+F233Y-12aa, T291A+G292A+T70C+I160G+S204L+V244S+T134I+M326Q+Y144W+G77T+I165L+A2925+5278 R+Y69M+G145H-12aa, T291A+G292A+T70C+I160G+S204L+V244S+T134I+M326Q+Y144W+G77T+I165L+A2925+5278 R+Y69M+G145D-12aa, T291A+G292A+T70C+I160G+S204L+V244S+T134I+M326Q+Y144W+G77T+I165L+A2925+5278 R+Y69M+A148T-12aa, T291A+G292A+T70C+I160G+S204L+V244S+T134I+M326Q+Y144W+G77T+I165L+A292S+S278 R+Y69M+A148Q-12aa, T291A+G292A+T70C+I160G+S204L+V244S+T134I+M326Q+Y144W+G77T+I165L+A2925+5278 R+Y69M+R152K-12aa, T291A+G292A+T70C+I160G+S204L+V244S+T134I+M326Q+Y144W+G77T+I165L+A2925+5278 R+Y69M-12aa, T291A+G292A+T70C+I160G+S204L+V244S+T134I+M326Q+Y144W+G77T+I165L+A292S+S278 R+Y69C-12aa, T291A+G292A+T70C+I160G+S204L+V244S+T134I+M326Q+Y144W+G77T+I165L+A292S+S278 R+Y69G-12aa, T291A+G292A+T70C+I160G+S204L+V244S+T134I+M326Q+Y144W+G77T+I165L+A2925+5278 R+Y69H-12aa, T291A+G292A+T70C+I160G+S204L+V244S+T134I+M326Q+Y144W+G77T+I165L+A2925+5278 R+Y69H-12aa, T291A+G292A+T70G+I160G+S204L+V2445+T134I+M326Q+Y144W+G77T+I165L+A2925+5278 R+Y69H-12aa, T291A+G292A+T70S+I160G+S204L+V2445+T134I+M326Q+Y144W+G77T+I165L+A292S+S278 R+Y69H-12aa, T291A+G292A+T70C+I160G+S204L+V244S+T134I+M326Q+Y144W+G77T+I165L+A2925+5278 R+Y69H+G140C-12aa, T291A+G292A+T70C+I160G+S204L+V244S+T134I+M326Q+Y144W+G77T+I165L+A2925+5278 R+Y69H+S141G-12aa, T291A+G292A+T70A+I160G+S204L+V244S+T134I+M326Q+Y144W+G77T+I165L+A292S+S278 R+Y69H+G140C-12aa, T291A+G292A+T70G+I160G+S204L+V2445+T134I+M326Q+Y144W+G77T+I165L+A2925+5278 R+Y69H+Y139S-12aa, T291A+G292A+T70G+I160G+S204L+V2445+T134I+M326Q+Y144W+G77T+I165L+A2925+5278 R+Y69H+G160T-12aa, T291A+G292A+T70G+I160G+S204L+V2445+T134I+M326Q+Y144W+G77T+I165L+A2925+5278 R+Y69H+G 160T+S292A-12aa, T291A+G292A+T70G+I160G+S204L+V2445+T134I+M326Q+Y144W+G77T+I165L+A2925+5278 R+Y69H+G160T+A291T-12aa, T291A+G292A+T70G+I160G+S204L+V2445+T134I+M326Q+Y144W+G77T+I165L+A2925+5278 R+Y69H+G160T+I134V-12aa, T291A+G292A+T70G+I160G+S204L+V2445+T134I+M326Q+Y144W+G77T+I165L+A2925+5278 R+Y69H+G160T+T106S-12aa, or T291A+G292A+T70G+I160G+S204L+V2445+T134I+M326Q+Y144W+G77T+I165L+A2925+5278 R+Y69H+G160T+Q171T-12aa.

Another aspect of the present disclosure provides a DNA molecule. The DNA molecule encodes any one of the above transaminase mutants.

Still another aspect of the present disclosure provides a recombinant plasmid. The recombinant plasmid contains any one of the above DNA molecules.

Further, the recombinant plasmid is pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, or pUC-19.

Yet another aspect of the present disclosure provides a host cell. The host cell contains the above recombinant plasmid.

Further the host cell includes a prokaryotic cell or a eukaryotic cell; preferably, the prokaryotic cell is *Escherichia coli* BL21-DE3 cell or *Escherichia coli* Rosetta-DE3 cell; and the eukaryotic cell is yeast.

Still yet another aspect of the present disclosure provides a method for producing a chiral amine. The method includes a step of using a transaminase to catalyze a ctransamination reaction of a ketone compound and an amino donor, and the transaminase is any one of the above transaminase mutants.
Further, the ketone compound is 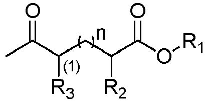 , where R1 is H, an unsubstituted or optionally halogen-substituted C1-C10 alkyl, an unsubstituted or optionally halogen-substituted C5-C10 cycloalkyl, and an unsubstituted or optionally halogen-substituted C5-C10 aryl or C5-C10 heteroaryl, R₂ is H, a halogen, and a C1-C10 alkyl unsubstituted or optionally substituted with the halogen, NH₃ or at least one group in NH₃ protected with Boc and Cbz, and R₃ is an unsubstituted or optionally halogen-substituted C6-12 aryl, and an unsubstituted or optionally halogen-substituted C3-6 heterocyclyl; and preferably, the ketone compound is

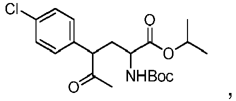

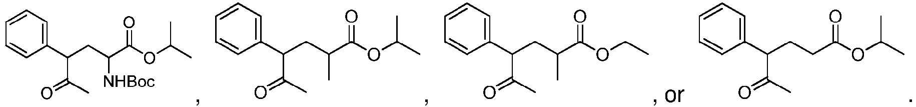

Further, the amino donor is isopropylamine or alanine, preferably the isopropylamine.

Further, in a reaction system in which the transaminase catalyzes the transamination reaction of the ketone compound and the amino donor, a pH is 7-11, preferably 8-10.5, and more preferably 10.5; preferably, in the reaction system in which the transaminase catalyzes the transamination reaction of the ketone compound and the amino donor, the temperature is 25-60°C, further preferably 30-55°C, and even more preferably 50°C; and preferably, a volume concentration of dimethyl sulfoxide in the reaction system the transaminase catalyzes the transamination reaction of the ketone compound and the amino donor is 0% to 50%.

The transaminase mutant of the present disclosure is based on the transaminase shown in SEQ ID NO:1, amino acid mutations and combination mutations thereof are performed on proteins to obtain mutants by means of rational design and directed evolution, so as to expand a substrate spectrum of enzymes, and improve the catalytic capacity of a large steric hindrance substrate; at the same time, the stability of the enzymes is improved, and a catalytic reaction may be performed at high solvent concentrations and high temperatures, such that the application of the mutant may increase a reaction rate, improve the stability of the enzymes, reduce a use amount of the enzymes, and reduce the difficulty of post-processing, thereby causing the mutant to be able to fit industrial production; in addition, the stereoselectivity of the transaminase mutant is also greatly improved, chiral amines may be produced more efficiently, and production costs and post-processing difficulties are reduced, such that the transaminase mutant is suitable for promotion of industrial production of the chiral amines.

### Detailed Description of the Embodiments

It is to be noted that the embodiments in the present disclosure and the features in the embodiments may be combined with one another without conflict. The present disclosure will be described below in detail with reference to the embodiments.

Transaminase is a type of protein-based biological catalysts. In an industrial production process, a certain organic solvent, a pressure, a temperature and other conditions that are easy to denature a protein are often required. Therefore, the biological catalyst used needs to have high tolerance in order to meet the needs of the industrial production. However, wild transaminase often on the one hand has low catalytic activity and narrow substrate spectra, and often has low tolerance to industrial demand conditions, thereby limiting wide application thereof.

The present disclosure seeks to perform amino acid mutations and combination mutations thereof on proteins to obtain mutants by means of rational design and directed evolution, so as to expand a substrate spectrum of enzymes, and improve the catalytic capacity of a large steric hindrance substrate; at the same time, the stability of the enzymes is improved, and a catalytic reaction may be performed at high solvent concentrations and high temperatures; and the stereoselectivity of a (1) chiral center in a structure is improved.

Rational transformation of an enzyme is based on a three-dimensional molecular structure of the enzyme to transform a substrate binding site, a coenzyme binding site, a surface and other parts of the enzyme, so as to change catalytic properties of the enzyme and improve the characteristics, such as activity and selectivity, of the enzyme. Directed evolution of the enzyme is a non-rational design of the protein, a special evolutionary condition is artificially created, a natural evolutionary mechanism is simulated, a gene is modified in vitro, and error-prone PCR, DNA shuffling and other technologies are applied, a new enzyme with expected properties is obtained in combination with an efficient screening system.

In the present disclosure, vitality screening of a substrate 1 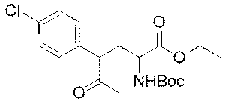 is performed on 150 natural transaminases and 190 transaminase mutants available in the present laboratory, and it is found that only the transaminase mutant MTTTEFANREIH(12aa)+G17V+Q40H+T66M+G69Y+H70T+L73A+V77G+A78I+K141S+K142T+R 143P+G144Y+Y130M+L148A+L151H+T152R+H153P+L163Q+A165I+R188L+T204S+S207I+F20 8R+K211H+T290S+A292G+K146R+E145G (Template, with an amino acid sequence being SEQ ID NO.1:
MTTTEFANREIH(12aa)MTTTEFANSNLVAVEPVAIREPTPPGSVIQYSEYELDRSHPLAGGVAWIEG EYVPADEARISIFDMGFYTSDATYTGIHVWHGNIFRLEDHLDRLLHGAARLKLETGMSREELAGIAK RCVSLSQLREAMVNITITRGYGSTPYGRDATKHRPQVYVYAIPYQWIFPPEEQIFGTSVIVPRHVRR AGLNTIDPTIKNFQWGDLSAAIREAHDRGARSAVLLDADNCVAEGPGFNVVLVKDGALVSPSRNAL PGITRKTVYEIAAAKGIETMLRDVTSSELYEADELMAVSTGGGVTPITSLDGEQVGNGEPGPITVAIR DRFWALMDEPSSLIEAIDY* (note, an amino acid position number in the present disclosure still corresponds to a position number of a transaminase of *Sciscionella sp.,* i.e., a 1st amino acid in SEQ ID NO.1 is the 1st "M" after 12aa, and so on); and correspondingly, a Template base sequence is SEQ ID NO.2 : 
ATGACCACCACCGAGTTTGCCAACAGGGAATTCCAT(12aa)ATGACCACCACCGAGTTTGCCAA
CAGCAATCTGGTGGCCGTGGAACCGGTTGCAATCCGTGAGCCTACCCCTCCGGGCAGCGTGA
TTCAGTACAGCGAGTACGAACTGGATCGCAGCCATCCGCTGGCAGGTGGCGTTGCCTGGATT
GAAGGCGAGTATGTTCCTGCCGATGAAGCCCGTATCAGCATCTTCGATATGGGCTTTTATACCA
GCGATGCGACCTATACCGGGATCCACGTTTGGCACGGCAATATCTTCCGCCTGGAAGACCACC
TGGACCGCCTGCTGCATGGTGCCGCACGTCTGAAACTGGAAACCGGTATGAGCCGCGAAGAA
CTGGCCGGCATTGCCAAGCGTTGTGTTAGCCTGAGCCAGCTGCGCGAAGCCATGGTGAACAT
CACCATTACCCGCGGCTATGGCAGCACCCCCTACGGAAGGGATGCGACCAAACATAGGCCCC
AGGTGTACGTGTATGCCATCCCGTATCAGTGGATCTTCCCTCCGGAGGAACAGATTTTCGGCA
 
CCAGCGTGATTGTGCCGCGTCATGTGCGCCGCGCCGGTCTCAATACCATCGACCCGACCATC
AAGAACTTTCAGTGGGGTGACCTGTCCGCCGCCATCCGTGAGGCCCATGATCGCGGCGCAC
GTAGCGCAGTTCTGCTGGATGCCGATAATTGCGTGGCCGAGGGTCCGGGCTTTAATGTGGTTC
TGGTGAAGGACGGCGCACTGGTGAGTCCGAGTCGTAACGCACTGCCGGGCATCACCCGCAA
AACCGTGTACGAAATCGCAGCAGCCAAAGGCATCGAAACCATGCTGCGCGACGTGACCAGCA
GTGAACTGTATGAGGCAGATGAGCTGATGGCCGTGAGCACCGGAGGTGGTGTGACCCCTATT
ACCAGCCTGGATGGCGAGCAGGTTGGCAACGGTGAGCCGGGCCCGATTACCGTGGCCATTC
GTGATCGCTTTTGGGCACTGATGGATGAGCCGAGCAGCCTGATTGAGGCCATTGACTATTAA)
 derived from *Sciscionella sp.* may catalyze a target substrate to obtain a product but has poor catalytic activity.

In order to expand the substrate spectrum of the enzymes, improve the catalytic capacity of the large steric hindrance substrate, and improve the stability of the enzymes, in the present disclosure, pET22b is used as an expression vector, a mutation site is introduced on the transaminase by means of site-directed mutagenesis, saturation mutations, error-prone PCR, combination mutations, etc., so as to obtain a plasmid containing a mutant gene; and BL21(DE3) is used as an expression strain, and a mutant protein is obtained under the induction of IPTG. Then activity testing is performed on the mutants, so as to select the mutants with improved activity.

Herein, site-directed mutagenesis: refers to the introduction of desired changes (usually characterizing changes in favorable directions) to the target DNA fragments (either genomes or plasmids) by polymerase chain reaction (PCR), including addition, deletion, point mutation, etc. of bases. Site-directed mutagenesis can rapidly and efficiently improve the properties and characterization of target proteins expressed by DNA, and is a very useful means in gene research..

The method of introducing site-directed mutation by whole plasmid PCR is simple and effective, and is a widely used method at present. The principle is as follows, a pair of primers containing mutation sites (forward and reverse), and the template plasmid is annealed, then "cycled extended" by polymerase, the so-called cyclic extension means that the polymerase extends the primers according to the template, and then returns to the 5' end of the primers after a circle, after cycles of repeated heating and annealing, this reaction is different from rolling circle amplification, will not form multiple tandem copies. The extension products of the forward primer and the reverse primer are annealed and paired to form a nicked open circular plasmid. Dpn I digests the extension product, since the original template plasmid is derived from conventional E. coli, subjected to dam methylation modification and sensitive to Dpn I, it is chopped, and the plasmid with the mutant sequence synthesized in vitro is not cut due to no methylation, so that the plasmid is successfully transformed in subsequent transformation, and clone of the mutant plasmid can be obtained.

The above mutant plasmid is transformed into *Escherichia coli* cell, and overexpressed in the *Escherichia coli.* Best conditions for the induction of transaminase expression are as follows: it is induced overnight at 25°C and 0.1mM IPTG. Then, a crude enzyme is obtained by a method of sonicating cells.

Reaction validation conditions include: preparing a substrate mother solution (dissolving a substrate in DMSO) and a PLP mother solution (dissolving 10 mg of pyridoxal 5-phosphate monohydrate in 1 mL of a buffer), sequentially adding a boric acid buffer, the PLP mother solution, a 6M isopropylamine hydrochloride solution, and the substrate mother solution in a 10 mL reactor, finally adding an enzyme (20% enzyme solution), performing a reaction in a shaker at 200rpm, adding acetonitrile in a reaction system after the reaction ends, performing well mixing through oscillation, then performing centrifugation for 3 min at 12000rpm, taking the supernatant and diluting it to a suitable multiple, and detecting activity through HPLC.

Preliminary modification is performed on the transaminase by means of rational methods of site-directed mutagenesis and single point saturation mutation in an earlier stage, and the substrate 1 is used to perform vitality verification: a reaction equation is as follows, and results are shown in Table 1.

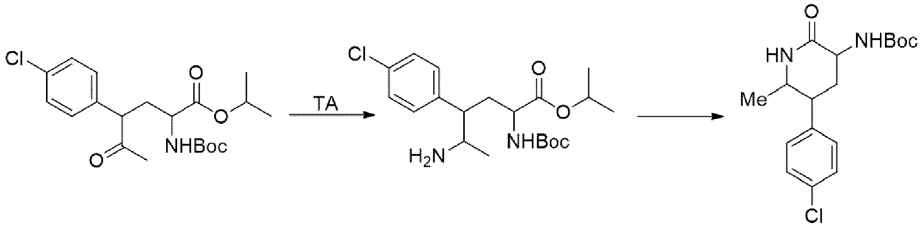

**Table 1**

| Mutant | Enzyme activity | Reaction condition |
|---|---|---|
| Template | + | A |
| T70A | + | A |
| Y75F | ++ | A |
| Y75A | - | A |
| G77V | - | A |
| G77A | + | A |
| Y144F | + | A |
| Y144A | + | A |
| M130Y | - | A |
| M130A | - | A |
| Q163L | + | A |
| Q163A | ++ | A |
| I165A | ++ | A |
| W164F | + | A |
| W164A | + | A |
| W200F | N.D. | A |
| W200A | - | A |
| G232A | - | A |
| G232V | - | A |
| F233Y | + | A |
| S290A | - | A |
| S290T | + | A |
| T291A | ++ | A |
| G292A | ++ | A |
| G292V | - | A |
| T291A+S290L | + | A |
| T291A+S290V | ++ | A |
| T291A+S290T | ++ | A |
| T291Q | ++ | A |
| T291E | - | A |
| T291V | + | A |
| T291F | - | A |
| T291T | + | A |
| T291I | + | A |
| T291K | + | A |
| T291C | ++ | A |
| T291Y | - | A |
| T291G | + | A |
| T291N | + | A |
| T291L | + | A |
| T291S | ++ | A |
| T291W | N.D. | A |
| T291R | - | A |
| T291H | - | A |
| T291P | - | A |
| T291M | ++ | A |
| T291A+G292A | +++ | A |
| T291A+G292L | + | A |
| T291A+G2921 | + | A |
| T291A+G292D | ++ | A |
| T291A+G292A+Y75T | ++ | A |
| T291A+G292A+G77E | +++ | A |
| T291A+G292A+M130I | +++ | A |
| T291A+G292A+M130L | +++ | A |
| T291A+G292A+Y144S | ++ | A |
| T291A+G292A+Y144G | ++ | A |
| T291A+G292A+Y144I | +++ | A |
| T291A+G292A+Y144V | +++ | A |
| T291A+G292A+Y144P | +++ | A |
| T291A+G292A+Y144L | +++ | A |
| T291A+G292A+Y144H | ++ | A |
| T291A+G292A+Q163S | +++ | A |
| T291A+G292A+Q163T | +++ | A |
| T291A+G292A+Q163L | ++ | A |
| T291A+G292A+Q163N | +++ | A |
| T291A+G292A+Q163V | ++ | A |
| T291A+G292A+W164F | +++ | A |
| T291A+G292A+W164R | ++ | A |
| T291A+G292A+W164L | ++ | A |
| T291A+G292A+W164T | ++ | A |
| T291A+G292A+I165A | ++ | A |
| T291A+G292A+I165V | +++ | A |
| T291A+G292A+I165L | ++ | A |
| T291A+G292A+W200G | ++ | A |
| T291A+G292A+W200H | - | A |
| T291A+G292A+L232F | +++ | A |
| T291A+G292A+F233W | ++ | A |
| T291A+G292A+F233Y | +++ | A |
| T291A+G292A+T70G | +++ | A |
| T291A+G292A+T70M | +++ | A |
| T291A+G292A+T70A | +++ | A |
| T291A+G292A+T70S | ++ | A |
| T291A+G292A+T70C | ++++ | A |
| T291A+G292A+W164Y | ++ | A |

Note:
1) Reaction condition A: 5wt enzyme, 2mg of PLP, 20% DMSO, 30eq of IPN, 500V, 0.1M Tris-Cl 9.0, 37°C, 40h.
2) N.D. indicates no product production detected; - represents 0-1%; + represents 1-5%; ++ represents 5-10%; +++ represents 10-15%; and ++++ represents >15%.

The above results indicate that the mutants modified by site-directed mutagenesis and directed evolution have acquired the ability to catalyze a substrate with large steric hindrance, and with a continuous and progressive increase in viability. In order to further improve the catalytic activity and stability of the mutants, the tolerance of the mutants is modified by means of saturation mutation and combination mutation and a directed screening method. An effect verification reaction equation is as follows, and results are shown in Table 2.

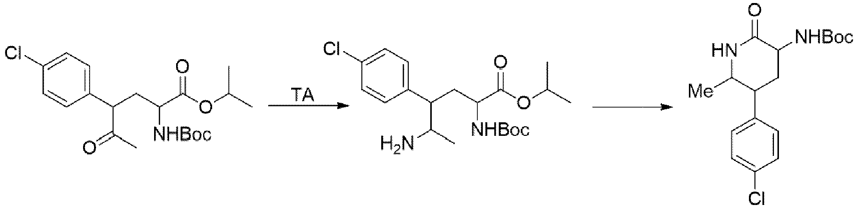

**Table 2**

| Mutant | Enzyme activity | Reaction condition |
|---|---|---|
| T291A+G292A+T70C | + | B |
| T291A+G292A+T70C+W164F | ++ | B |
| T291A+G292A+T70C+F233Y | ++ | B |
| T291A+G292A+T70C+I165V | +++ | B |
| T291A+G292A+T70C+Y144M+G145N | +++ | B |
| T291A+G292A+T70C+Y144M+G145S | +++ | B |
| T291A+G292A+T70C+G145L | ++ | B |
| T291A+G292A+T70C+Y69L | ++ | B |
| T291A+G292A+T70C+T142G | ++ | B |
| T291A+G292A+T70C+I160G | +++++ | B |
| T291A+G292A+T70C+I160T | +++ | B |
| T291A+G292A+T70C+I160S | +++ | B |
| T291A+G292A+T70C+I160V | +++ | B |
| T291A+G292A+T70C+I160C | ++ | B |
| T291A+G292A+T70C+S204L | ++++ | B |
| T291A+G292A+T70C+S204M | ++++ | B |
| T291A+G292A+T70C+V244G | +++ | B |
| T291A+G292A+T70C+V244R | +++ | B |
| T291A+G292A+T70C+V244Y | +++ | B |
| T291A+G292A+T70C+V244S | ++++ | B |
| T291A+G292A+T70C+V244K | +++ | B |
| T291A+G292A+T70C+V244M | ++++ | B |
| T291A+G292A+T70C+I160G+H151A | +++++ | B |
| T291A+G292A+T70C+I160G+H151D | +++++ | B |
| T291A+G292A+T70C+I160G+H151Y | +++++ | B |
| T291A+G292A+T70C+I160G+H151Q | +++++ | B |
| T291A+G292A+T70C+I160G | ++ | C |
| T291A+G292A+T70C+I160G+S204L | +++ | C |
| T291A+G292A+T70C+I160G+V244S | +++ | C |
| T291A+G292A+T70C+I160G+S204L+V244S | +++ | C |
| T291A+G292A+T70C+I160G+T134I | ++ | C |
| T291A+G292A+T70C+I160G+A242S | ++ | C |
| T291A+G292A+T70C+I160G+A242Q | ++ | C |
| T291A+G292A+T70C+I160G+T270G | ++ | C |
| T291A+G292A+T70C+I160G+T270H | ++ | C |
| T291A+G292A+T70C+I160G+S278L | ++ | C |
| T291A+G292A+T70C+I160G+S278R | +++ | C |
| T291A+G292A+T70C+I160G+S278K | ++ | C |
| T291A+G292A+T70C+I160G+E282C | + | C |
| T291A+G292A+T70C+I160G+E282R | ++ | C |
| T291A+G292A+T70C+I160G+E282K | + | C |
| T291A+G292A+T70C+I160G+M326Q | +++ | C |
| T291A+G292A+T70C+I160G+V17S | ++ | C |
| T291A+G292A+T70C+I160G+A56S | ++ | C |
| T291A+G292A+T70C+I160G+F64L | ++ | C |
| T291A+G292A+T70C+I160G+P167A | ++ | C |
| T291A+G292A+T70C+I160G+P167K | ++ | C |
| T291A+G292A+T70C+I160G+R216E | +++ | C |
| T291A+G292A+T70C+I160G+R216K | ++ | C |
| T291A+G292A+T70C+I160G+S204L+V244S+T134I | ++++ | C |
| T291A+G292A+T70C+I160G+S204L+V244S+M326Q | +++ | C |
| T291A+G292A+T70C+I160G+S204L+V244S+T134I | ++++ | D |
| T291A+G292A+T70C+I160G+S204L+V244S+T134I+M326Q | ++++ | D |
| T291A+G292A+T70C+I160G+S204L+V244S+T134I+M326Q | +++ | E |
| T291A+G292A+T70C+I160G+S204L+V244S+T134I+M326Q+Y158I | +++++ | E |
| T291A+G292A+T70C+I160G+S204L+V244S+T134I+M326Q+Y158V | +++++ | E |
| T291A+G292A+T70C+I160G+S204L+V244S+T134I+M326Q+R216N | +++ | E |
| T291A+G292A+T70C+I160G+S204L+V244S+T134I+M326Q+R216K | +++ | E |

Note:
1) Reaction condition B: 2wt enzyme, 4mg of PLP, 30% DMSO, 30eq of IPN, 200V, 0.1M boric acid 10.0, 50°C, 16h.
2) Reaction condition C: .5wt enzyme, 4g/L of PLP, 30% DMSO, 30eq of IPN, 100V, 0.1M boric acid 10.0, 50°C, 16h.
3) Reaction condition D: 0.5wt enzyme, 1g/L of PLP, 30% DMSO, 30eq of IPN, 100V, 0.1M boric acid 10.5, 55°C, 16h.
4) Reaction condition E: 0.1wt enzyme, 1g/L of PLP, 50% DMSO, 5eq of IPN, 100V, 0.2M boric acid 10.5, 60°C, 16h.
5) N.D. indicates no product production detected; - represents 0-10%; + represents 10-20%; ++ represents 20-50 % ; +++ represents 50-80% ; and ++++ represents 80-90%; and +++++ represents >90%.

The mutants of which tolerance is modified is subjected to enzyme activity validation of the substrate 1, the stability of the mutants in terms of high temperature resistance is significantly improved, an optimal temperature of Template is 37°C, optimal temperatures of the mutants such as a mutant T291A+G292A+T70C, a mutant T291A+G292A+T70C+I160G, etc. are increased to 50°C, and optimal temperatures of the mutants T291A+G292A+T70C+I160G+S204L+V244S+T134I+M326Q and T291A+G292A+T70C+I160G+S204L+V244S+T134I+M326Q+Y158V are increased to 60°C; at the same time, the stability of the mutants in a solvent is also significantly improved, and an optimal reaction DMSO concentration increases from 20% to 50%; and furthermore, the enzyme activity is also continuously and progressively improved, and the enzyme activity of the mutant T291A+G292A+T70C+I160G+S204L+V244S+T134I+M326Q+Y158V is greatly improved compared to that of Template. In order to improve the stereoselectivity of the mutants, the selectivity of the mutants is modified by means of evolution methods of saturation mutation, combination mutation, and error-prone PCR and a directed screening method. An effect verification reaction equation is as follows, and results are shown in Table 3.

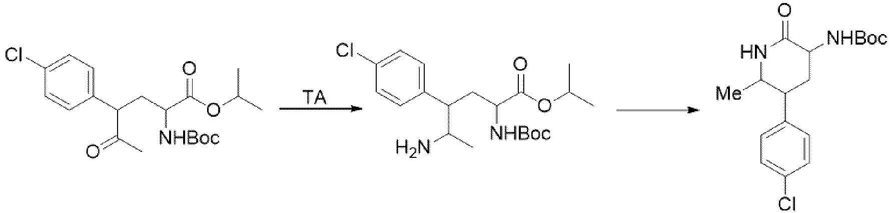

**Table 3**

| Mutant | Enz yme activ ity | ee val ue | Reac tion condi tion |
|---|---|---|---|
| T291A+G292A+T70C+I160G+S204L+V244S+T134I+M326Q | +++ ++ | N. D. | D |
| T291A+G292A+T70C+I160G+S204L+V244S+T134I+M326Q+G77V | + | ** | F |
| T291A+G292A+T70C+I160G+S204L+V244S+T134I+M326Q+W200A | + | * | F |
| T291A+G292A+T70C+I160G+S204L+V244S+T134I+M326Q+Y144L | +++ ++ | N. D. | F |
| T291A+G292A+T70C+I160G+S204L+V244S+T134I+M326Q+Y144L+G77T | +++ ++ | *** | F |
| T291A+G292A+T70C+I160G+S204L+V244S+T134I+M326Q+Y144L+G77L | +++ | *** | F |
| T291A+G292A+T70C+I160G+S204L+V244S+T134I+M326Q+A292G | +++ | * | F |
| | ++ | | |
| T291A+G292A+T70C+I160G+S204L+V244S+T134I+M326Q+A292G+G77T | +++ ++ | ** | F |
| T291A+G292A+T70C+I160G+S204L+V244S+T134I+M326Q+W200A+A56P | ++ | * | F |
| T291A+G292A+T70C+I160G+S204L+V244S+T134I+M326Q+W200A+A56P+ N132A | + | *** | F |
| T291A+G292A+T70C+I160G+S204L+V244S+T134I+M326Q+W200A+A56P+ G77S | ++ | ** | F |
| T291A+G292A+T70C+I160G+S204L+V244S+T134I+M326Q+W200A+A56P+ G77T | ++ | *** | F |
| T291A+G292A+T70C+I160G+S204L+V244S+T134I+M326Q+W200A+A56P+ Y144F | + | ** | F |
| T291A+G292A+T70C+I160G+S204L+V244S+T134I+M326Q+Y144L+G77T | +++ | *** * | G |
| T291A+G292A+T70C+I160G+S204L+V244S+T134I+M326Q+Y144L+G77T-1 2aa | +++ + | *** | G |
| T291A+G292A+T70C+I160G+S204L+V244S+T134I+M326Q+A292G+G77T-1 2aa | ++ | *** | G |
| T291A+G292A+T70C+I160G+S204L+V244S+T134I+M326Q+Y144L+G77T+L 144W-12aa | +++ | *** * | G |
| T291A+G292A+T70C+I160G+S204L+V244S+T134I+M326Q+Y144L+G77T+L 144P-12aa | ++ | *** * | G |
| T291A+G292A+T70C+I160G+S204L+V244S+T1341+M326Q+Y144L+G77T+ N132R-12aa | | *** ** | G |
| T291A+G292A+T70C+I160G+S204L+V244S+T134I+M326Q+Y144L+G77T+I 165L-12aa | +++ + | *** * | G |
| T291A+G292A+T70C+I160G+S204L+V244S+T134I+M326Q+Y144L+G77T+I 165H-12aa | + | *** * | G |
| T291A+G292A+T70C+I160G+S204L+V244S+T134I+M326Q+Y144W+G77T+ I165L-12aa | +++ ++ | *** * | G |
| T291A+G292A+T70C+I160G+S204L+V244S+T134I+M326Q+Y144W+G77T+ I165H-12aa | +++ + | *** * | G |
| T291A+G292A+T70C+I160G+S204L+V244S+T134I+M326Q+Y144W+G77T+ I165L-12aa | +++ | *** * | H |
| T291A+G292A+T70C+I160G+S204L+V244S+T134I+M326Q+Y144W+G77T+ I165L+I78V-12aa | +++ + | *** * | H |
| T291A+G292A+T70C+I160G+S204L+V244S+T134I+M326Q+Y144W+G77T+ I165L+A291I-12aa | ++ | *** ** | H |
| T291A+G292A+T70C+I160G+S204L+V244S+T134I+M326Q+Y144W+G77T+ I165L+A292S-12aa | +++ | *** ** | H |
| T291A+G292A+T70C+I160G+S204L+V244S+T134I+M326Q+Y144W+G77T+ I165L+A292S+I78H-12aa | +++ | *** ** | H |
| T291A+G292A+T70C+I160G+S204L+V244S+T134I+M326Q+Y144W+G77T+ I165L+A292S+S278R-12aa | +++ + | *** ** | H |
| T291A+G292A+T70C+I160G+S204L+V244S+T134I+M326Q+Y144W+G77T+ I165L+A292S+S278R-12aa | +++ | *** ** | L |
| T291A+G292A+T70C+I160G+S204L+V244S+T134I+M326Q+Y144W+G77T+ I165L+A2925+S278R+T142V-12aa | +++ | *** ** | L |
| T291A+G292A+T70C+I160G+S204L+V244S+T134I+M326Q+Y144W+G77T+ I165L+A292S+S278R+G145H-12aa | +++ | *** ** | L |
| T291A+G292A+T70C+I160G+S204L+V244S+T134I+M326Q+Y144W+G77T+ I165L+A292S+S278R+G145D-12aa | +++ + | *** ** | L |
| T291A+G292A+T70C+I160G+S204L+V244S+T134I+M326Q+Y144W+G77T+ I165L+A292S+S278R+A148T-12aa | +++ | *** ** | L |
| T291A+G292A+T70C+I160G+S204L+V244S+T134I+M326Q+Y144W+G77T+ I165L+A292S+S278R+A148Q-12aa | +++ | *** ** | L |
| T291A+G292A+T70C+I160G+S204L+V244S+T134I+M326Q+Y144W+G77T+ I165L+A292S+S278R+R152K-12aa | +++ | *** ** | L |
| T291A+G292A+T70C+I160G+S204L+V244S+T134I+M326Q+Y144W+G77T+ I165L+A292S+S278R+R152T-12aa | +++ | *** ** | L |
| T291A+G292A+T70C+I160G+S204L+V244S+T134I+M326Q+Y144W+G77T+ I165L+A292S+S278R+F233Y-12aa | +++ | *** ** | L |
| T291A+G292A+T70C+I160G+S204L+V244S+T134I+M326Q+Y144W+G77T+ I165L+A292S+S278R+Y69M+G145H-12aa | +++ + | *** ** | L |
| T291A+G292A+T70C+I160G+S204L+V244S+T134I+M326Q+Y144W+G77T+ I165L+A292S+S278R+Y69M+G145D-12aa | +++ ++ | *** * | L |
| T291A+G292A+T70C+I160G+S204L+V244S+T134I+M326Q+Y144W+G77T+ I165L+A292S+S278R+Y69M+A148T-12aa | +++ + | *** ** | L |
| T291A+G292A+T70C+I160G+S204L+V244S+T134I+M326Q+Y144W+G77T+ I165L+A292S+S278R+Y69M+A148Q-12aa | +++ + | *** ** | L |
| T291A+G292A+T70C+I160G+S204L+V244S+T134I+M326Q+Y144W+G77T+ I165L+A292S+S278R+Y69M+R152K-12aa | +++ + | *** ** | L |
| T291A+G292A+T70C+I160G+S204L+V244S+T134I+M326Q+Y144W+G77T+ I165L+A292S+S278R+Y69M-12aa | +++ | *** ** | L |
| T291A+G292A+T70C+I160G+S204L+V244S+T134I+M326Q+Y144W+G77T+ I165L+A292S+S278R+Y69C-12aa | ++ | *** *** | L |
| T291A+G292A+T70C+I160G+S204L+V244S+T134I+M326Q+Y144W+G77T+ I165L+A292S+S278R+Y69G-12aa | ++ | *** *** | L |
| T291A+G292A+T70C+I160G+S204L+V244S+T134I+M326Q+Y144W+G77T+ I165L+A292S+S278R+Y69H-12aa | ++ | *** *** | L |
| T291A+G292A+T70C+I160G+S204L+V244S+T134I+M326Q+Y144W+G77T+ I165L+A292S+S278R+Y69H-12aa | + | *** *** | M |
| T291A+G292A+T70G+I160G+S204L+V244S+T134I+M326Q+Y144W+G77T+ I165L+A292S+S278R+Y69H-12aa | +++ + | *** *** | M |
| T291A+G292A+T70G+I160G+S204L+V244S+T134I+M326Q+Y144W+G77T+ I165L+A292S+S278R+Y69H-12aa | +++ | *** *** | M |
| T291A+G292A+T70C+I160G+S204L+V244S+T134I+M326Q+Y144W+G77T+ I165L+A292S+S278R+Y69H+G140C-12aa | ++ | *** ** | M |
| T291A+G292A+T70C+I160G+S204L+V244S+T134I+M326Q+Y144W+G77T+ I165L+A292S+S278R+Y69H+S141G-12aa | +++ | *** *** | M |
| T291A+G292A+T70A+I160G+S204L+V244S+T134I+M326Q+Y144W+G77T+ I165L+A292S+S278R+Y69H+G140C-12aa | +++ | *** *** | M |
| T291A+G292A+T70G+I160G+S204L+V244S+T134I+M326Q+Y144W+G77T+ I165L+A292S+S278R+Y69H+Y139S-12aa | +++ + | *** *** | M |
| T291A+G292A+T70G+I160G+S204L+V244S+T134I+M326Q+Y144W+G77T+ I165L+A292S+S278R+Y69H+G160T-12aa | +++ + | *** ** | M |
| T291A+G292A+T70G+I160G+S204L+V244S+T134I+M326Q+Y144W+G77T+ I165L+A292S+S278R+Y69H+G160T+S292A-12aa | +++ ++ | *** ** | M |
| T291A+G292A+T70G+I160G+S204L+V244S+T134I+M326Q+Y144W+G77T+ I165L+A292S+S278R+Y69H+G160T+A291T-12aa | +++ + | *** ** | M |
| T291A+G292A+T70G+I160G+S204L+V244S+T134I+M326Q+Y144W+G77T+ I165L+A292S+S278R+Y69H+G160T+I134V-12aa | +++ ++ | *** ** | M |
| T291A+G292A+T70G+I160G+S204L+V244S+T134I+M326Q+Y144W+G77T+ I165L+A292S+S278R+Y69H+G160T+T106S-12aa | +++ ++ | *** *** | M |
| T291A+G292A+T70G+I160G+S204L+V244S+T134I+M326Q+Y144W+G77T+ I165L+A292S+S278R+Y69H+G160T+Q171T-12aa | +++ ++ | *** * | M |

| | | | |
|---|---|---|---|
| "-12aa" indicates the removal of 12 amino acids from a front end of a protein. | | | |

Note: 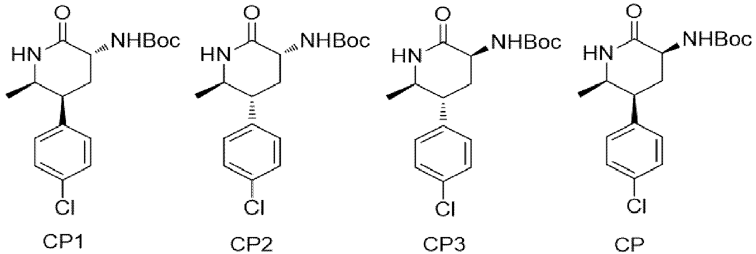
1) Transaminase mutant ee value, ee value=((CP1+CP)-(CP2+CP3))/(CP+CP1+CP2+CP3)
2) Reaction condition D: 0.5wt enzyme, 1g/L of PLP, 30% DMSO, 30eq of isopropylamine hydrochloride (IPN), 100V, 0.1M boric acid 10.5, 55°C, 16h.
3) Reaction condition F: 7wt enzyme, 1g/L of PLP, 20% DMSO, 30eq of IPN, 500V, 0.2M boric acid 10.5, 60°C, 16h.
4) Reaction condition G: 0.1wt enzyme, 1g/L of PLP, 30% DMSO, 10eq of IPN, 100V, 0.2M boric acid 10.5, 60°C, 16h.
5) Reaction condition H: 0.3wt enzyme, 1g/L of PLP, 30% DMSO, 10eq of IPN, 100V, 0.2M boric acid 10.5, 60°C, 16h.
6) Reaction condition L: 0.3wt enzyme, 1g/L of PLP, 40% DMSO, 10eq of IPN, 100V, 0.2M boric acid 10.5, 60°C, 16h.
7) Reaction condition M: 0.3wt enzyme, 1g/L of PLP, 40% DMSO, 10eq of IPN, 100V, 0.2M boric acid 10.5, 55°C, 16h.
8) N.D. indicates no product production detected; - represents 0-10%; + represents 10-20%; ++ represents 20-50 % ; +++ represents 50-80% ; and ++++ represents 80-90%; and +++++ represents >90%.
8) N.D. represents 0; * represents 0-10%; ** represents 10-50%; *** represents 50-80%; **** represents 80-90% ; ***** represents 90-95% ; and ****** represents >95%.

The mutants of which selectivity is modified is subjected to enzyme activity validation of the substrate 1, the selectivity of the mutants is continuously gradually improved, ee values of transaminase mutants T291A+G292A+T70C+I160G+S204L+V244S+T134I+M326Q+Y144L+G77L and T291A+G292A+T70C+I160G+S204L+V244S+T134I+M326Q+A292S increase to more than 70%, ee values of transaminase mutants T291A+G292A+T70C+I160G+S204L+V244S+T134I+M326Q+Y144L+G77T, T291A+G292A+T70C+I160G+S204L+V244S+T134I+M326Q+Y144L+G77T+L144W-12aa, and T291A+G292A+T70C+I160G+S204L+V244S+T134I+M326Q+Y144W+G77T+I165L-12aa increase to more than 80%, ee values of transaminase mutants T291A+G292A+T70C+I160G+S204L+V244S+T134I+M326Q+Y144W+G77T+I165L+A292S-12aa and T291A+G292A+T70C+I160G+S204L+V244S+T134I+M326Q+Y144W+G77T+I165L+A292S+S278 R-12aa increase to more than 90%, and a ee value of transaminase mutant T291A+G292A+T70C+I160G+S204L+V244S+T134I+M326Q+Y144W+G77T+I165L+A292S+S278 R+Y69H+T70G+G160T+T106S-12aa increases to more than 95%.

Computer simulation analysis is performed on a three-dimensional structure of the transaminase (Template) by using software, most of mutated sites are located near an active center (active center sites mainly include sites such as T70, Y144, A292, Y69, I160, etc.), and the binding of a substrate and an enzyme may be improved after mutation, thereby improving selectivity and catalytic efficiency.

A typical implementation of the present disclosure provides a transaminase mutant. An amino acid sequence of the transaminase mutant is obtained by mutation occurred in an amino acid sequence as shown in SEQ ID NO:1, and the mutation includes at least T291A; or the amino acid sequence of the transaminase mutant has a mutation site in the mutated amino acid sequence, and has more than 80% of identity with the mutated amino acid sequence, preferably has 85% of identity, more preferably has 90% of identity, further preferably has 95% of identity, and further preferably has 99% of identity. Preferably, the mutation includes T291A and at least one of the following mutation sites: V17S, A56S, A56P, F64L, Y69L, Y69M, Y69C, Y69G, T70A, T70G, T70M, T70C, T70S, Y75F, G77E, G77S, G77V, G77T, G77L, I78H, T106S, M130I, M130L, N132W, N132A, T134I, T134A, T134V, Y139S, G140C, S141G, K142G, K142V, Y144I, Y144V, Y144P, Y144L, Y144M, Y144F, E145N, E145S, E145L, E145H, E145D, A148T, A148Q, H151A, H151D, H151Y, H151Q, R152K, R152T, Y158I, Y158V, I160G, I160T, I160S, I160V, I160C, I160T, Q163S, Q163T, Q163N, Q163A, W164F, I165L, I165H, I165A, I165V, P167A, P167K, Q171T, W200A, S204L, S204M, R216E, R216K, R216N, G232F, F233Y, A242S, A242Q, V244G, V244R, V244Y, V244S, V244K, V244M, T270G, T270H, S278L, S278R, S278K, E282C, E282R, E282K, T291A, T291Q, T291C, T291S, T291M, T291T, G292A, G292S, or M326Q.

A term "identity" used herein has the meaning generally known in the field, and those skilled in the art are also familiar with rules and standards for measuring identity between different sequences. The sequences defined by the present disclosure with different degrees of identity must also have the improved tolerance of the transaminase to the organic solvent. In the above implementation, preferably the amino acid sequence of the transaminase mutant has the above identity and has or encodes the amino acid sequence with the improved tolerance to the organic solvent. Those skilled in the art may obtain such mutant sequences under the teaching of the disclosed content of the present disclosure.

Preferably, the mutation includes at least one of the following mutation site combinations: T291A+S290L, T291A+S290V, T291A+S290T, T291A+G292A, T291A+G292L, T291A+G292I, T291A+G292D, T291A+G292A+Y75T, T291A+G292A+G77E, T291A+G292A+M130I, T291A+G292A+M130L, T291A+G292A+Y144S, T291A+G292A+Y144G, T291A+G292A+Y144I, T291A+G292A+Y144V, T291A+G292A+Y144P, T291A+G292A+Y144L, T291A+G292A+Y144H, T291A+G292A+Q163S, T291A+G292A+Q163T, T291A+G292A+Q163L, T291A+G292A+Q163N, T291A+G292A+Q163V, T291A+G292A+W164F, T291A+G292A+W164R, T291A+G292A+W164L, T291A+G292A+W164T, T291A+G292A+I165A, T291A+G292A+I165V, T291A+G292A+I165L, T291A+G292A+W200G, T291A+G292A+T70C, T291A+G292A+T70C+W164F, T291A+G292A+T70C+F233Y, T291A+G292A+T70C+I165V, T291A+G292A+T70C+Y144M+G145N, T291A+G292A+T70C+Y144M+G145S, T291A+G292A+T70C+G145L, T291A+G292A+T70C+Y69L, T291A+G292A+T70C+T142G, T291A+G292A+T70C+I160G, T291A+G292A+T70C+I160T, T291A+G292A+T70C+I160S, T291A+G292A+T70C+I160V, T291A+G292A+T70C+I160C, T291A+G292A+T70C+S204L, T291A+G292A+T70C+S204M, T291A+G292A+T70C+V244G, T291A+G292A+T70C+V244R, T291A+G292A+T70C+V244Y, T291A+G292A+T70C+V244S, T291A+G292A+T70C+V244K, T291A+G292A+T70C+V244M, T291A+G292A+T70C+I160G+H151A, T291A+G292A+T70C+I160G+H151D, T291A+G292A+T70C+I160G+H151Y, T291A+G292A+T70C+I160G+H151Q, T291A+G292A+T70C+I160G, T291A+G292A+T70C+I160G+S204L, T291A+G292A+T70C+I160G+V244S, T291A+G292A+T70C+I160G+S204L+V244S, T291A+G292A+T70C+I160G+T134I, T291A+G292A+T70C+I160G+A242S, T291A+G292A+T70C+I160G+A242Q, T291A+G292A+T70C+I160G+T270G, T291A+G292A+T70C+I160G+T270H, T291A+G292A+T70C+I160G+S278L, T291A+G292A+T70C+I160G+S278R, T291A+G292A+T70C+I160G+S278K, T291A+G292A+T70C+I160G+E282C, T291A+G292A+T70C+I160G+E282R, T291A+G292A+T70C+I160G+E282K, T291A+G292A+T70C+I160G+M326Q, T291A+G292A+T70C+I160G+V17S, T291A+G292A+T70C+I160G+A56S, T291A+G292A+T70C+I160G+F64L, T291A+G292A+T70C+I160G+P167A, T291A+G292A+T70C+I160G+P167K, T291A+G292A+T70C+I160G+R216E, T291A+G292A+T70C+I160G+R216K, T291A+G292A+T70C+I160G+S204L+V244S+T134I, T291A+G292A+T70C+I160G+S204L+V244S+M326Q, T291A+G292A+T70C+I160G+S204L+V244S+T134I, T291A+G292A+T70C+I160G+S204L+V244S+T134I+M326Q, T291A+G292A+T70C+I160G+S204L+V244S+T134I+M326Q, T291A+G292A+T70C+I160G+S204L+V244S+T134I+M326Q+Y158I, T291A+G292A+T70C+I160G+S204L+V244S+T134I+M326Q+Y158V, T291A+G292A+T70C+I160G+S204L+V244S+T134I+M326Q+R216N, T291A+G292A+T70C+I160G+S204L+V244S+T134I+M326Q+R216K, T291A+G292A+L232F, T291A+G292A+F233W, T291A+G292A+F233Y, T291A+G292A+T70G, T291A+G292A+T70M, T291A+G292A+T70A, T291A+G292A+T70S, T291A+G292A+T70C, T291A+G292A+W164Y, T291A+G292A+T70C+I160G+S204L+V244S+T134I+M326Q+G77V, T291A+G292A+T70C+I160G+S204L+V244S+T134I+M326Q+W200A, T291A+G292A+T70C+I160G+S204L+V244S+T134I+M326Q+Y144L, T291A+G292A+T70C+I160G+S204L+V244S+T134I+M326Q+Y144L+G77T, T291A+G292A+T70C+I160G+S204L+V244S+T134I+M326Q+Y144L+G77L, T291A+G292A+T70C+I160G+S204L+V244S+T134I+M326Q+A292G, T291A+G292A+T70C+I160G+S204L+V244S+T134I+M326Q+A292G+G77T, T291A+G292A+T70C+I160G+S204L+V244S+T134I+M326Q+W200A+A56P, T291A+G292A+T70C+I160G+S204L+V244S+T134I+M326Q+W200A+A56P+N132A, T291A+G292A+T70C+I160G+S204L+V244S+T134I+M326Q+W200A+A56P+G77S, T291A+G292A+T70C+I160G+S204L+V244S+T134I+M326Q+W200A+A56P+G77T, T291A+G292A+T70C+I160G+S204L+V244S+T134I+M326Q+W200A+A56P+Y144F, T291A+G292A+T70C+I160G+S204L+V244S+T134I+M326Q+Y144L+G77T, T291A+G292A+T70C+I160G+S204L+V244S+T134I+M326Q+Y144L+G77T-12aa, T291A+G292A+T70C+I160G+S204L+V244S+T134I+M326Q+A292G+G77T-12aa, T291A+G292A+T70C+I160G+S204L+V244S+T134I+M326Q+Y144L+G77T+L144W-12aa, T291A+G292A+T70C+I160G+S204L+V244S+T134I+M326Q+Y144L+G77T+L144P-12aa, T291A+G292A+T70C+I160G+S204L+V244S+T134I+M326Q+Y144L+G77T+N132R-12aa, T291A+G292A+T70C+I160G+S204L+V244S+T134I+M326Q+Y144L+G77T+I165L-12aa, T291A+G292A+T70C+I160G+S204L+V244S+T134I+M326Q+Y144L+G77T+I165H-12aa, T291A+G292A+T70C+I160G+S204L+V244S+T134I+M326Q+Y144W+G77T+I165L-12aa, T291A+G292A+T70C+I160G+S204L+V244S+T134I+M326Q+Y144W+G77T+I165H-12aa, T291A+G292A+T70C+I160G+S204L+V244S+T134I+M326Q+Y144W+G77T+I165L-12aa, T291A+G292A+T70C+I160G+S204L+V244S+T134I+M326Q+Y144W+G77T+I165L+I78V-12aa, T291A+G292A+T70C+I160G+S204L+V244S+T134I+M326Q+Y144W+G77T+I165L+A291I-12aa, T291A+G292A+T70C+I160G+S204L+V244S+T134I+M326Q+Y144W+G77T+I165L+A292S-12aa, T291A+G292A+T70C+I160G+S204L+V244S+T134I+M326Q+Y144W+G77T+I165L+A292S+I78H-12aa, T291A+G292A+T70C+I160G+S204L+V244S+T134I+M326Q+Y144W+G77T+I165L+A292S+S278 R-12aa, T291A+G292A+T70C+I160G+S204L+V244S+T134I+M326Q+Y144W+G77T+I165L+A292S+S278 R-12aa, T291A+G292A+T70C+I160G+S204L+V244S+T134I+M326Q+Y144W+G77T+I165L+A292S+S278 R+T142V-12aa, T291A+G292A+T70C+I160G+S204L+V244S+T134I+M326Q+Y144W+G77T+I165L+A2925+5278 R+G 145H-12aa, T291A+G292A+T70C+I160G+S204L+V244S+T134I+M326Q+Y144W+G77T+I165L+A292S+S278 R+G 145D-12aa, T291A+G292A+T70C+I160G+S204L+V244S+T134I+M326Q+Y144W+G77T+I165L+A292S+S278 R+A148T-12aa, T291A+G292A+T70C+I160G+S204L+V244S+T134I+M326Q+Y144W+G77T+I165L+A292S+S278 R+A 148Q-12aa, T291A+G292A+T70C+I160G+S204L+V244S+T134I+M326Q+Y144W+G77T+I165L+A292S+S278 R+R152K-12aa, T291A+G292A+T70C+I160G+S204L+V244S+T134I+M326Q+Y144W+G77T+I165L+A2925+5278 R+R152T-12aa, T291A+G292A+T70C+I160G+S204L+V244S+T134I+M326Q+Y144W+G77T+I165L+A2925+5278 R+F233Y-12aa, T291A+G292A+T70C+I160G+S204L+V244S+T134I+M326Q+Y144W+G77T+I165L+A2925+5278 R+Y69M+G145H-12aa, T291A+G292A+T70C+I160G+S204L+V244S+T134I+M326Q+Y144W+G77T+I165L+A2925+5278 R+Y69M+G145D-12aa, T291A+G292A+T70C+I160G+S204L+V244S+T134I+M326Q+Y144W+G77T+I165L+A2925+5278 R+Y69M+A148T-12aa, T291A+G292A+T70C+I160G+S204L+V244S+T134I+M326Q+Y144W+G77T+I165L+A292S+S278 R+Y69M+A148Q-12aa, T291A+G292A+T70C+I160G+S204L+V244S+T134I+M326Q+Y144W+G77T+I165L+A2925+5278 R+Y69M+R152K-12aa, T291A+G292A+T70C+I160G+S204L+V244S+T134I+M326Q+Y144W+G77T+I165L+A2925+5278 R+Y69M-12aa, T291A+G292A+T70C+I160G+S204L+V244S+T134I+M326Q+Y144W+G77T+I165L+A292S+S278 R+Y69C-12aa, T291A+G292A+T70C+I160G+S204L+V244S+T134I+M326Q+Y144W+G77T+I165L+A292S+S278 R+Y69G-12aa, T291A+G292A+T70C+I160G+S204L+V244S+T134I+M326Q+Y144W+G77T+I165L+A2925+5278 R+Y69H-12aa, T291A+G292A+T70C+I160G+S204L+V244S+T134I+M326Q+Y144W+G77T+I165L+A2925+5278 R+Y69H-12aa, T291A+G292A+T70G+I160G+S204L+V2445+T134I+M326Q+Y144W+G77T+I165L+A2925+5278 R+Y69H-12aa, T291A+G292A+T70G+I160G+S204L+V2445+T134I+M326Q+Y144W+G77T+I165L+A2925+5278 R+Y69H-12aa, T291A+G292A+T70C+I160G+S204L+V244S+T134I+M326Q+Y144W+G77T+I165L+A2925+5278 R+Y69H+G140C-12aa, T291A+G292A+T70C+I160G+S204L+V244S+T134I+M326Q+Y144W+G77T+I165L+A292S+S278 R+Y69H+S141G-12aa, T291A+G292A+T70A+I160G+S204L+V2445+T1341+M326Q+Y144W+G77T+I165L+A292S+S278 R+Y69H+G140C-12aa, T291A+G292A+T70G+I160G+S204L+V2445+T134I+M326Q+Y144W+G77T+I165L+A2925+5278 R+Y69H+Y139S-12aa, T291A+G292A+T70G+I160G+S204L+V2445+T134I+M326Q+Y144W+G77T+I165L+A2925+5278 R+Y69H+G160T-12aa, T291A+G292A+T70G+I160G+S204L+V2445+T134I+M326Q+Y144W+G77T+I165L+A2925+5278 R+Y69H+G 160T+S292A-12aa, T291A+G292A+T70G+I160G+S204L+V2445+T134I+M326Q+Y144W+G77T+I165L+A2925+5278 R+Y69H+G160T+A291T-12aa, T291A+G292A+T70G+I160G+S204L+V2445+T134I+M326Q+Y144W+G77T+I165L+A2925+5278 R+Y69H+G160T+I134V-12aa, T291A+G292A+T70G+I160G+S204L+V244S+T134I+M326Q+Y144W+G77T+I165L+A292S+S278 R+Y69H+G160T+T106S-12aa, or T291A+G292A+T70G+I160G+S204L+V244S+T134I+M326Q+Y144W+G77T+I165L+A292S+S278 R+Y69H+G160T+Q171T-12aa.

A typical implementation of the present disclosure provides a DNA molecule. The DNA molecule encodes the above transaminase mutant tolerant to an organic solvent. The above transaminase mutant encoded by the DNA molecule has good organic solvent tolerance and high pH tolerance, and has high soluble expression characteristics and high activity characteristics.

The above DNA molecules of the present disclosure may also exist in the form of "expression cassette". "Expression cassette" refers to a linear or circular nucleic acid molecule that encompasses DNA and RNA sequences capable of directing the expression of a particular nucleotide sequence in an appropriate host cell. Generally, it includes a promoter operably linked to a target nucleotide, optionally operably linked to a termination signal and/or other regulatory elements. The expression cassette may also include sequences required for correct translation of nucleotide sequences. The coding region usually encodes the target protein, but it also encodes the target functional RNA, such as antisense RNA or untranslated RNA, in the sense or antisense direction. An expression cassette containing a target polynucleotide sequence may be chimeric, meaning that at least one of the components is heterologous to at least one of the other components. The expression cassette can also exist naturally, but can be obtained by effective recombination for heterologous expression.

According to an exemplary embodiment of the present disclosure, a recombinant plasmid is provided. The recombinant plasmid contains any of the DNA molecules described above. The DNA molecule in the recombinant plasmid is placed in position on the recombinant plasmid so that the DNA molecule can be correctly and smoothly replicated, transcribed or expressed.

Although the term "contain" is used herein to define a DNA molecule as defined above, it does not mean that additional sequences not functionally related thereto may be added at either end of the DNA sequence. Those skilled in the art know that, in order to meet the requirements of the recombination operation, it is necessary to add appropriate restriction sites of restriction endonuclease at both ends of the DNA sequence, or to additionally add start codon, terminator codon and the like, and therefore, these situations will not be truly covered if the closed expression is used to define them.

The term "plasmid" used in the disclosure includes any plasmid, cosmid, bacteriophage or agrobacterium binary nucleic acid molecule in double-stranded or single-stranded linear or circular form, which is preferably a recombinant expression plasmid, either prokaryotic expression plasmid or eukaryotic expression plasmid, but preferably prokaryotic expression plasmids. In some implementations, the recombinant plasmid is selected from pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, or pUC-19. More preferably, the above recombinant plasmid is pET-22b(+).

A typical implementation of the present disclosure provides a host cell, and the host cell contains any one of the above recombinant plasmids. The host cell suitable for the present disclosure includes, but is not limited to, a prokaryotic cell or a eukaryotic cell. Preferably, the prokaryotic cell is eubacteria, such as gram-negative bacteria or gram-positive bacteria. More preferably, the prokaryotic cell is *Escherichia coli* BL21 cell or *Escherichia coli* DH5α competent cell; and the eukaryotic cell is yeast.

A typical implementation of the present disclosure provides a method for producing a chiral amine. The method includes a step of using a transaminase to catalyze a transamination reaction of a ketone compound and an amino donor, and the transaminase is any one of the above transaminase mutants tolerant to an organic solvent. Since the transaminase mutant of the present disclosure has high capability of expanding a substrate spectrum of enzymes and improving the catalytic capacity of a large steric hindrance substrate; and at the same time, the stability is improved, and a catalytic reaction may be performed at high solvent concentrations and high temperatures, such that the application of the mutant may increase a reaction rate, improve the stability of the enzymes, reduce a use amount of the enzymes, and reduce the difficulty of post-processing, thereby causing the mutant to be able to fit industrial production.

Further, the ketone compound is 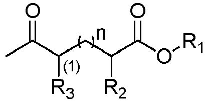 , where R₁ is H, an unsubstituted or optionally halogen-substituted C1-C10 alkyl, an unsubstituted or optionally halogen-substituted C5-C10 cycloalkyl, and an unsubstituted or optionally halogen-substituted C5-C10 aryl or C5-C10 heteroaryl, R₂ is H, a halogen, and a C1-C10 alkyl unsubstituted or optionally substituted with the halogen, NH₃ or at least one group in NH₃ protected with Boc and Cbz, and R₃ is an unsubstituted or optionally halogen-substituted C6-12 aryl, and an unsubstituted or optionally halogen-substituted C3-6 heterocyclyl.

Preferably, the ketone compound is 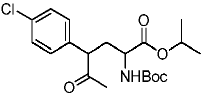 (substrate 1), 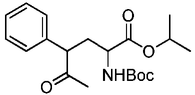 (substrate 2), 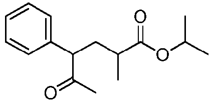 (substrate 3), 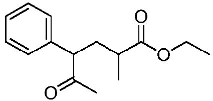 (substrate 4), or 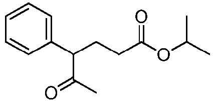 (substrate 5).

In a typical implementation of the present disclosure, the amino donor is isopropylamine or alanine, preferably the isopropylamine.

In a reaction system in which the transaminase of the present disclosure is applied to catalyze a transamination reaction of the ketone compound and the amino donor, a pH is 7-10.5, preferably 8-10, more preferably 9-10, that is to say, the pH may be a value optionally selected from 7 to 10.5, such as 7, 7.5, 8, 8, 8.6, 9, 10, 10.5, etc. A temperature of the reaction system in which the transaminase is used to catalyze the transamination reaction of the ketone compound and the amino donor is 25-60°C, more preferably 30-55°C, further preferably 40-50°C, in other words, the temperature may be a value optionally selected from 25°C to 60°C, such as 30, 31, 32, 35, 37, 38, 39, 40, 42, 45, 48, 50, 51, 52, 55, etc. A volume concentration of the dimethyl sulfoxide in the reaction system in which the transaminase is used to catalyze the transamination reaction of the ketone compound and the amino donor is 0%-50%, for example, 10%, 15%, 18%, 20%, 30%, 35%, 38%, 40%, 42%, 48%, 49%, etc.

It is known by those skilled in the art that many modifications may be made to the present disclosure without departing from spirit of the present disclosure, and such modifications also fall within a scope of the present disclosure. In addition, the following experimental methods are conventional methods unless otherwise specified, and experimental materials used may be easily obtained from commercial companies unless otherwise specified.

The beneficial effects of the present disclosure are further described below with reference to embodiments.

### Embodiment 1

Template and mutants catalyzed a ketone compound to produce a chiral amine, activity validation was performed under reaction conditions M, results showed that the Template had poor catalytic activity to a substrate, while Template mutants had good catalytic activity, and a ee value of a mutant T291A+G292A+I160G+S204L+V244S+T134I+M326Q+Y144W+G77T+I165L+A292S+S278R+Y69 H+T70G+G160T+T106S-12aa increased to more than 90%. Through the modification of the present disclosure, the Template mutants obtained large catalytic activity to a substrate with large steric hindrance, the activity of some mutants was greatly improved, and the stereoselectivity of the mutant was also greatly improved, thereby expanding a substrate spectrum. A reaction equation was as follows, and results were shown in Table 4.

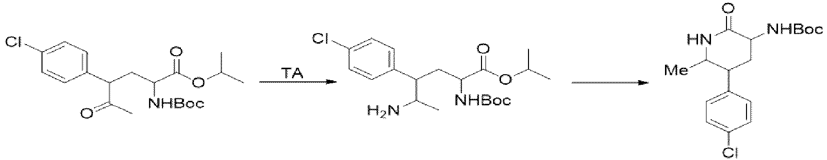

**Table 4**

| Mutant | Enz yme activ ity | ee val ue | Reac tion condi tion |
|---|---|---|---|
| Template | - | N. D. | M |
| T291A+G292A+T70G+I160G+S204L+V244S+T134I+M326Q+Y144W+G77T+ I165L+A292S+S278R+Y69H-12aa | +++ + | *** *** | M |
| T291A+G292A+T70S+I160G+S204L+V244S+T134I+M326Q+Y144W+G77T+ I165L+A292S+S278R+Y69H-12aa | +++ | *** *** | M |
| T291A+G292A+T70C+I160G+S204L+V244S+T134I+M326Q+Y144W+G77T+ I165L+A292S+S278R+Y69H+G140C-12aa | ++ | *** ** | M |
| T291A+G292A+T70C+I160G+S204L+V244S+T134I+M326Q+Y144W+G77T+ I165L+A292S+S278R+Y69H+S141G-12aa | +++ | *** *** | M |
| T291A+G292A+T70A+I160G+S204L+V244S+T134I+M326Q+Y144W+G77T+ I165L+A292S+S278R+Y69H+G140C-12aa | +++ | *** *** | M |
| T291A+G292A+T70G+I160G+S204L+V244S+T134I+M326Q+Y144W+G77T+ I165L+A292S+S278R+Y69H+Y139S-12aa | +++ + | *** *** | M |
| T291A+G292A+T70G+I160G+S204L+V244S+T134I+M326Q+Y144W+G77T+ I165L+A292S+S278R+Y69H+G160T-12aa | +++ + | *** ** | M |
| T291A+G292A+T70G+I160G+S204L+V244S+T134I+M326Q+Y144W+G77T+ I165L+A292S+S278R+Y69H+G160T+S292A-12aa | +++ ++ | *** ** | M |
| T291A+G292A+T70G+I160G+S204L+V244S+T134I+M326Q+Y144W+G77T+ I165L+A292S+S278R+Y69H+G160T+A291T-12aa | +++ + | *** ** | M |
| T291A+G292A+T70G+I160G+S204L+V244S+T134I+M326Q+Y144W+G77T+ I165L+A292S+S278R+Y69H+G160T+I134V-12aa | +++ ++ | *** ** | M |
| T291A+G292A+T70G+I160G+S204L+V244S+T134I+M326Q+Y144W+G77T+ I165L+A292S+S278R+Y69H+G160T+T106S-12aa | +++ ++ | *** *** | M |

Note:
1) Reaction condition M: 0.3wt enzyme, 1g/L of PLP, 40% DMSO, 10eq of IPN, 100V, 0.2M boric acid with pH=10.5, 55°C, 16h.
2) N.D. indicated no product production detected; - represented 0-10%; + represented 10-20%; ++ represented 20-50%; +++ represented 50-80%; and ++++ represented 80-90%; and +++++ represented >90%.
3) N.D. represented 0; * represented 0-10%; ** represented 10-50%; *** represented 50-80%; **** represented 80-90%; ***** represented 90-95%; and ****** represented >95%.

### Embodiment 2

Template and mutants catalyzed a ketone compound to produce a chiral amine, activity validation was performed under reaction conditions N, results showed that the Template had no catalytic activity to a substrate, while Template mutants had good catalytic activity. Through the modification of the present disclosure, the Template mutants expanded a catalytic substrate spectrum. A reaction equation was as follows, and results were shown in Table 5.

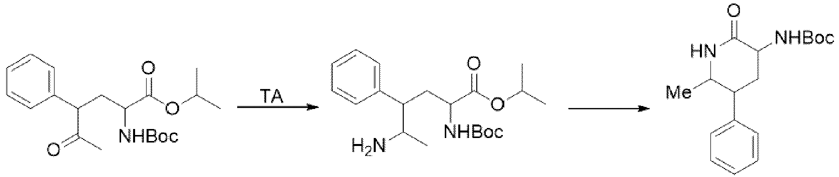

**Table 5**

| Mutant | Enzyme activity | Reaction condition |
|---|---|---|
| Template | N.D. | N |
| T291A+G292A+F233Y | | N |
| T291A+G292A+T70C | + | N |
| T291A+G292A+T70C+I160G | ++ | N |
| T291A+G292A+T70C+S204L | ++ | N |
| T291A+G292A+T70C+S204M | ++ | N |
| T291A+G292A+T70C+I160G+S204L | +++ | N |
| T291A+G292A+T70C+I160G+V244S | ++ | N |
| T291A+G292A+T70C+I160G+S204L+V244S | +++ | N |
| T291A+G292A+T70C+I160G+S204L+V244S+T134I | ++++ | N |
| T291A+G292A+T70C+I160G+S204L+V244S+M326Q | ++++ | N |
| T291A+G292A+T70C+I160G+S204L+V244S+T134I+M326Q | +++++ | N |
| T291A+G292A+T70C+I160G+S204L+V244S+T134I+M326Q+Y158I | ++++++ | N |
| T291A+G292A+T70C+I160G+S204L+V244S+T134I+M326Q+Y158V | ++++++ | N |

Note:
Reaction condition M: 1wt enzyme, 1g/L of PLP, 20% DMSO, 10eq of IPN, 100V, 0.2M boric acid with pH=10.5, 50°C, 16h.
N.D. indicated no product production detected; - represented 0-10%; + represented 10-20%; ++ represented 20-50%; +++ represented 50-80%; ++++ represented 80-90%; +++++ represented 90%-95%; and ++++++ represented >95%.

### Embodiment 3

Template and mutants catalyzed a ketone compound to produce a chiral amine, activity validation was performed under reaction conditions N, results showed that the Template had no catalytic activity to a substrate, while Template mutants had good catalytic activity. Through the modification of the present disclosure, the Template mutants expanded a catalytic substrate spectrum. A reaction equation was as follows, and results were shown in Table 6.

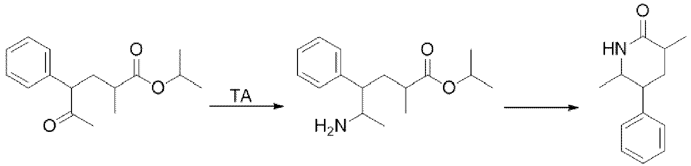

**Table 6**

| Mutant | Enzyme activity | Reaction condition |
|---|---|---|
| Template | N.D. | N |
| T291A+G292A+T70C+I160G+V244S | ++ | N |
| T291A+G292A+T70C+I160G+S204L+V244S | +++ | N |
| T291A+G292A+T70C+I160G+S204L+V244S+T134I | ++ | N |
| T291A+G292A+T70C+I160G+S204L+V244S+M326Q | +++ | N |
| T291A+G292A+T70C+I160G+S204L+V244S+T134I+M326Q | ++++ | N |
| T291A+G292A+T70C+I160G+S204L+V244S+T134I+M326Q+Y158I | ++++ | N |
| T291A+G292A+T70C+I160G+S204L+V244S+T134I+M326Q+Y158V | ++++++ | N |

Note:
Reaction condition M: 1wt enzyme, 1g/L of PLP, 20% DMSO, 10eq of IPN, 100V, 0.2M boric acid with pH=10.5, 50°C, 16h.
N.D. indicated no product production detected; - represented 0-10%; + represented 10-20%; ++ represented 20-50%; +++ represented 50-80%; ++++ represented 80-90%; +++++ represented 90%-95%; and ++++++ represented >95%.

### Embodiment 4

Template and mutants catalyzed a ketone compound to produce a chiral amine, activity validation was performed under reaction conditions N, results showed that the Template had no catalytic activity to a substrate, while Template mutants had good catalytic activity. Through the modification of the present disclosure, the Template mutants expanded a catalytic substrate spectrum. A reaction equation was as follows, and results were shown in Table 7.

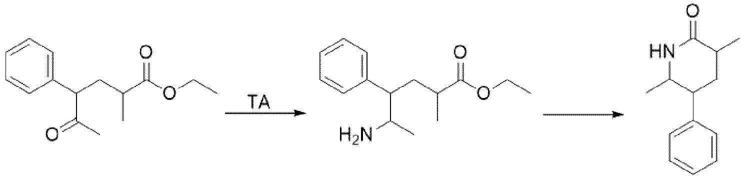

**Table 7**

| Mutant | Enzy me activi ty | Reac tion condi tion |
|---|---|---|
| Template | N.D. | N |
| T291A | + | N |
| G292A | + | N |
| T291A+G292A | ++ | N |
| T291A+G292A+T70C | ++ | N |
| T291A+G292A+T70C+I160G | +++ | N |
| T291A+G292A+T70C+I160G+S204L+V244S+T134I | +++ + | N |
| T291A+G292A+T70C+I160G+S204L+V244S+M326Q | +++ + | N |
| T291A+G292A+T70C+I160G+S204L+V244S+T134I+M326Q | +++ | N |
| T291A+G292A+T70C+I160G+S204L+V244S+T134I+M326Q+Y158V | +++ ++ | N |
| T291A+G292A+T70C+I160G+S204L+V244S+T134I+M326Q+Y144L+G77T | ++ | N |
| T291A+G292A+T70C+I160G+S204L+V244S+T134I+M326Q+Y144W+G77T+I165 L-12aa | +++ + | N |
| T291A+G292A+T70C+I160G+S204L+V244S+T134I+M326Q+Y144W+G77T+I165 H-12aa | +++ | N |
| T291A+G292A+T70C+I160G+S5204L+V244S+T134I+M326Q+Y144W+G77T+I165 L+A292S+I78H-12aa | +++ | N |
| T291A+G292A+T70C+I160G+S5204L+V244S+T134I+M326Q+Y144W+G77T+I165 L+A292S+S278R-12aa | +++ + | N |
| T291A+G292A+T70C+I160G+S204L+V244S+T134I+M326Q+Y144W+G77T+I165 L+A292S+S278R+Y69M+G 145D-12aa | ++ | N |
| T291A+G292A+T70C+I160G+S204L+V244S+T134I+M326Q+Y144W+G77T+I165 L+A292S+S278R+Y69M+A148T-12aa | +++ | N |
| T291A+G292A+T70G+I160G+S204L+V244S+T134I+M326Q+Y144W+G77T+I165 L+A292S+S278R+Y69H+G160T+S292A-12aa | +++ ++ | N |
| T291A+G292A+T70G+I160G+S204L+V244S+T134I+M326Q+Y144W+G77T+I165 | +++ | N |
| L+A292S+S278R+Y69H+G160T+T106S-12aa | + | |

Note:
Reaction condition M: 1wt enzyme, 1g/L of PLP, 20% DMSO, 10eq of IPN, 100V, 0.2M boric acid with pH=10.5, 50°C, 16h.
2) N.D. indicated no product production detected; - represented 0-20%; + represented 20-40%; ++ represented 40-60%; +++ represented 60-80%; and ++++ represented 80-90%; and +++++ represented 90-100%.

### Embodiment 5

Template and mutants catalyzed a ketone compound to produce a chiral amine, activity validation was performed under reaction conditions N, results showed that the Template had no catalytic activity to a substrate, while Template mutants had good catalytic activity. Through the modification of the present disclosure, the Template mutants expanded a catalytic substrate spectrum. A reaction equation was as follows, and results were shown in Table 8.

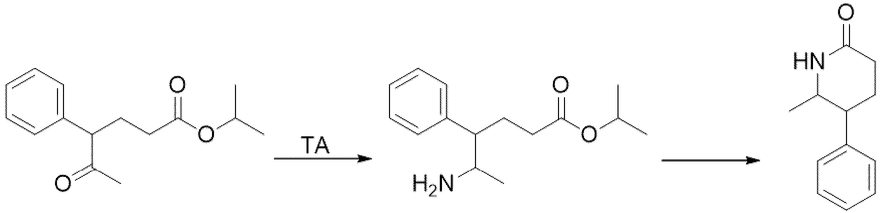

**Table 8**

| Mutant | Enzy me activi ty | Reac tion condi tion |
|---|---|---|
| Template | N.D. | N |
| T291A+G292A+T70C+I160G+S204L+V244S+M326Q | +++ + | N |
| T291A+G292A+T70C+I160G+S204L+V244S+T134I+M326Q | +++ | N |
| T291A+G292A+T70C+I160G+S204L+V244S+T134I+M326Q+Y158V | +++ ++ | N |
| T291A+G292A+T70G+I160G+S204L+V244S+T134I+M326Q+Y144W+G77T+I165 L+A292S+S278R+Y69H+G160T+S292A-12aa | +++ + | N |
| T291A+G292A+T70G+I160G+S204L+V244S+T134I+M326Q+Y144W+G77T+I165 L+A292S+S278R+Y69H+G160T+T106S-12aa | +++ ++ | N |

Note:
Reaction condition M: 1wt enzyme, 1g/L of PLP, 20% DMSO, 10eq of IPN, 100V, 0.2M boric acid with pH=10.5, 50°C, 16h.
2) N.D. indicated no product production detected; - represented 0-10%; + represented 10-20%; ++ represented 20-50%; +++ represented 50-80%; and ++++ represented 80-90%; and +++++ represented >90%.

From the above descriptions, it might be seen that, the following technical effects were implemented in the above embodiments of the present disclosure. The transaminase mutant of the present disclosure expanded a substrate spectrum of enzymes and improved the catalytic capacity of a large steric hindrance substrate; and at the same time, the stability was improved, and a catalytic reaction might be performed at high solvent concentrations and high temperatures, such that the application of the mutant might increase a reaction rate, improve the stability of the enzymes, reduce a use amount of the enzymes, and reduce the difficulty of post-processing, thereby causing the mutant to be able to fit industrial production.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure all fall within the scope of protection of the present disclosure.

## Claims

1. A transaminase mutant, wherein the transaminase mutant is obtained by mutation occurred in an amino acid sequence shown in SEQ ID NO: 1, and the mutation at least comprises: T291A; or an amino acid sequence of the transaminase mutant has the mutation site in the mutated amino acid sequence, and has more than 80% of the identity with the mutated amino acid sequence, preferably 85% of identity, more preferably 90% of identity, further preferably 95% of identity, and further preferably 99% of identity.

2. The transaminase mutant according to claim 1, wherein the mutation comprises T291A and at least one of the following mutation sites: V17S, A56S, A56P, F64L, Y69L, Y69M, Y69C, Y69G, T70A, T70G, T70M, T70C, T70S, Y75F, G77E, G77S, G77V, G77T, G77L, I78H, T106S, M130I, M130L, N132W, N132A, T134I, T134A, T134V, Y139S, G140C, S141G, K142G, K142V, Y144I, Y144V, Y144P, Y144L, Y144M, Y144F, E145N, E145S, E145L, E145H, E145D, A148T, A148Q, H151A, H151D, H151Y, H151Q, R152K, R152T, Y158I, Y158V, I160G, I160T, I160S, I160V, I160C, I160T, Q163S, Q163T, Q163N, Q163A, W164F, I165L, I165H, I165A, I165V, P167A, P167K, Q171T, W200A, S204L, S204M, R216E, R216K, R216N, G232F, F233Y, A242S, A242Q, V244G, V244R, V244Y, V244S, V244K, V244M, T270G, T270H, S278L, S278R, S278K, E282C, E282R, E282K, T291A, T291Q, T291C, T291S, T291M, T291T, G292A, G292S and M326Q.

3. The transaminase mutant according to claim 2, wherein the mutation further comprises at least one of the following mutation site combinations: T291A+S290L, T291A+S290V, T291A+S290T, T291A+G292A, T291A+G292L, T291A+G292I, T291A+G292D, T291A+G292A+Y75T, T291A+G292A+G77E, T291A+G292A+M130I, T291A+G292A+M130L, T291A+G292A+Y144S, T291A+G292A+Y144G, T291A+G292A+Y144I, T291A+G292A+Y144V, T291A+G292A+Y144P, T291A+G292A+Y144L, T291A+G292A+Y144H, T291A+G292A+Q163S, T291A+G292A+Q163T, T291A+G292A+Q163L, T291A+G292A+Q163N, T291A+G292A+Q163V, T291A+G292A+W164F, T291A+G292A+W164R, T291A+G292A+W164L, T291A+G292A+W164T, T291A+G292A+I165A, T291A+G292A+I165V, T291A+G292A+I165L, T291A+G292A+W200G, T291A+G292A+T70C, T291A+G292A+T70C+W164F, T291A+G292A+T70C+F233Y, T291A+G292A+T70C+I165V, T291A+G292A+T70C+Y144M+G145N, T291A+G292A+T70C+Y144M+G145S, T291A+G292A+T70C+G145L, T291A+G292A+T70C+Y69L, T291A+G292A+T70C+T142G, T291A+G292A+T70C+I160G, T291A+G292A+T70C+I160T, T291A+G292A+T70C+I160S, T291A+G292A+T70C+I160V, T291A+G292A+T70C+I160C, T291A+G292A+T70C+S204L, T291A+G292A+T70C+S204M, T291A+G292A+T70C+V244G, T291A+G292A+T70C+V244R, T291A+G292A+T70C+V244Y, T291A+G292A+T70C+V244S, T291A+G292A+T70C+V244K, T291A+G292A+T70C+V244M, T291A+G292A+T70C+I160G+H151A, T291A+G292A+T70C+I160G+H151D, T291A+G292A+T70C+I160G+H151Y, T291A+G292A+T70C+I160G+H151Q, T291A+G292A+T70C+I160G, T291A+G292A+T70C+I160G+S204L, T291A+G292A+T70C+I160G+V244S, T291A+G292A+T70C+I160G+S204L+V244S, T291A+G292A+T70C+I160G+T134I, T291A+G292A+T70C+I160G+A242S, T291A+G292A+T70C+I160G+A242Q, T291A+G292A+T70C+I160G+T270G, T291A+G292A+T70C+I160G+T270H, T291A+G292A+T70C+I160G+S278L, T291A+G292A+T70C+I160G+S278R, T291A+G292A+T70C+I160G+S278K, T291A+G292A+T70C+I160G+E282C, T291A+G292A+T70C+I160G+E282R, T291A+G292A+T70C+I160G+E282K, T291A+G292A+T70C+I160G+M326Q, T291A+G292A+T70C+I160G+V17S, T291A+G292A+T70C+I160G+A56S, T291A+G292A+T70C+I160G+F64L, T291A+G292A+T70C+I160G+P167A, T291A+G292A+T70C+I160G+P167K, T291A+G292A+T70C+I160G+R216E, T291A+G292A+T70C+I160G+R216K, T291A+G292A+T70C+I160G+S204L+V244S+T134I, T291A+G292A+T70C+I160G+S204L+V244S+M326Q, T291A+G292A+T70C+I160G+S204L+V244S+T134I, T291A+G292A+T70C+I160G+S204L+V244S+T134I+M326Q, T291A+G292A+T70C+I160G+S204L+V244S+T134I+M326Q, T291A+G292A+T70C+I160G+S204L+V244S+T134I+M326Q+Y158I, T291A+G292A+T70C+I160G+S204L+V244S+T134I+M326Q+Y158V, T291A+G292A+T70C+I160G+S204L+V244S+T134I+M326Q+R216N, T291A+G292A+T70C+I160G+S204L+V244S+T134I+M326Q+R216K, T291A+G292A+L232F, T291A+G292A+F233W, T291A+G292A+F233Y, T291A+G292A+T70G, T291A+G292A+T70M, T291A+G292A+T70A, T291A+G292A+T70S, T291A+G292A+T70C, T291A+G292A+W164Y, T291A+G292A+T70C+I160G+S204L+V244S+T134I+M326Q+G77V, T291A+G292A+T70C+I160G+S204L+V244S+T134I+M326Q+W200A, T291A+G292A+T70C+I160G+S204L+V244S+T134I+M326Q+Y144L, T291A+G292A+T70C+I160G+S204L+V244S+T134I+M326Q+Y144L+G77T, T291A+G292A+T70C+I160G+S204L+V244S+T134I+M326Q+Y144L+G77L, T291A+G292A+T70C+I160G+S204L+V244S+T134I+M326Q+A292G, T291A+G292A+T70C+I160G+S204L+V244S+T134I+M326Q+A292G+G77T, T291A+G292A+T70C+I160G+S204L+V244S+T134I+M326Q+W200A+A56P , T291A+G292A+T70C+I160G+S204L+V244S+T134I+M326Q+W200A+A56P+N132A, T291A+G292A+T70C+I160G+S204L+V244S+T134I+M326Q+W200A+A56P+G77S, T291A+G292A+T70C+I160G+S204L+V244S+T134I+M326Q+W200A+A56P+G77T, T291A+G292A+T70C+I160G+S204L+V244S+T134I+M326Q+W200A+A56P+Y144F, T291A+G292A+T70C+I160G+S204L+V244S+T134I+M326Q+Y144L+G77T, T291A+G292A+T70C+I160G+S204L+V244S+T134I+M326Q+Y144L+G77T-12aa, T291A+G292A+T70C+I160G+S204L+V244S+T134I+M326Q+A292G+G77T-12aa, T291A+G292A+T70C+I160G+S204L+V244S+T134I+M326Q+Y144L+G77T+L144W-12aa, T291A+G292A+T70C+I160G+S204L+V244S+T134I+M326Q+Y144L+G77T+L144P-12aa, T291A+G292A+T70C+I160G+S204L+V244S+T134I+M326Q+Y144L+G77T+N132R-12aa, T291A+G292A+T70C+I160G+S204L+V244S+T134I+M326Q+Y144L+G77T+I165L-12aa, T291A+G292A+T70C+I160G+S204L+V244S+T134I+M326Q+Y144L+G77T+I165H-12aa, T291A+G292A+T70C+I160G+S204L+V244S+T134I+M326Q+Y144W+G77T+I165L-12aa, T291A+G292A+T70C+I160G+S204L+V244S+T134I+M326Q+Y144W+G77T+I1165H-12aa, T291A+G292A+T70C+I160G+S204L+V244S+T134I+M326Q+Y144W+G77T+I165L-12aa, T291A+G292A+T70C+I160G+S204L+V244S+T134I+M326Q+Y144W+G77T+I165L+I78V-12aa, T291A+G292A+T70C+I160G+S204L+V244S+T134I+M326Q+Y144W+G77T+I1165L+A291I-12aa, T291A+G292A+T70C+I160G+S204L+V244S+T134I+M326Q+Y144W+G77T+I165L+A292S-12aa, T291A+G292A+T70C+I160G+S204L+V244S+T134I+M326Q+Y144W+G77T+I165L+A292S+I78H-12aa, T291A+G292A+T70C+I160G+S204L+V244S+T134I+M326Q+Y144W+G77T+I165L+A292S+S278 R-12aa, T291A+G292A+T70C+I160G+S204L+V244S+T134I+M326Q+Y144W+G77T+I165L+A292S+S278 R-12aa, T291A+G292A+T70C+I160G+S204L+V244S+T134I+M326Q+Y144W+G77T+I165L+A292S+S278 R+T142V-12aa, T291A+G292A+T70C+I160G+S204L+V244S+T134I+M326Q+Y144W+G77T+I165L+A2925+5278 R+G145H-12aa, T291A+G292A+T70C+I160G+S204L+V244S+T134I+M326Q+Y144W+G77T+I165L+A292S+S278 R+G145D-12aa, T291A+G292A+T70C+I160G+S204L+V244S+T134I+M326Q+Y144W+G77T+I165L+A292S+S278 R+A148T-12aa, T291A+G292A+T70C+I160G+S204L+V244S+T134I+M326Q+Y144W+G77T+I165L+A292S+S278 R+A148Q-12aa, T291A+G292A+T70C+I160G+S204L+V244S+T134I+M326Q+Y144W+G77T+I165L+A292S+S278 R+R152K-12aa, T291A+G292A+T70C+I160G+S204L+V244S+T134I+M326Q+Y144W+G77T+I165L+A2925+5278 R+R152T-12aa, T291A+G292A+T70C+I160G+S204L+V244S+T134I+M326Q+Y144W+G77T+I165L+A2925+5278 R+F233Y-12aa, T291A+G292A+T70C+I160G+S204L+V244S+T134I+M326Q+Y144W+G77T+I165L+A2925+5278 R+Y69M+G145H-12aa, T291A+G292A+T70C+I160G+S204L+V244S+T134I+M326Q+Y144W+G77T+I165L+A2925+5278 R+Y69M+G145D-12aa, T291A+G292A+T70C+I160G+S204L+V244S+T134I+M326Q+Y144W+G77T+I165L+A2925+5278 R+Y69M+A148T-12aa, T291A+G292A+T70C+I160G+S204L+V244S+T134I+M326Q+Y144W+G77T+I165L+A292S+S278 R+Y69M+A148Q-12aa, T291A+G292A+T70C+I160G+S204L+V244S+T134I+M326Q+Y144W+G77T+I165L+A2925+5278 R+Y69M+R152K-12aa, T291A+G292A+T70C+I160G+S204L+V244S+T134I+M326Q+Y144W+G77T+I165L+A2925+5278 R+Y69M-12aa, T291A+G292A+T70C+I160G+S204L+V244S+T134I+M326Q+Y144W+G77T+I165L+A292S+S278 R+Y69C-12aa, T291A+G292A+T70C+I160G+S204L+V244S+T134I+M326Q+Y144W+G77T+I165L+A292S+S278 R+Y69G-12aa, T291A+G292A+T70C+I160G+S204L+V244S+T134I+M326Q+Y144W+G77T+I165L+A2925+5278 R+Y69H-12aa, T291A+G292A+T70C+I160G+S204L+V244S+T134I+M326Q+Y144W+G77T+I165L+A2925+5278 R+Y69H-12aa, T291A+G292A+T70G+I160G+S204L+V244S+T134I+M326Q+Y144W+G77T+I165L+A292S+S278 R+Y69H -12aa, T291A+G292A+T70S+I160G+S204L+V244S+T134I+M326Q+Y144W+G77T+I165L+A292S+S278 R+Y69H -12aa, T291A+G292A+T70C+I160G+S204L+V244S+T134I+M326Q+Y144W+G77T+I165L+A292S+S278 R+Y69H+G140C-12aa, T291A+G292A+T70C+I160G+S204L+V244S+T134I+M326Q+Y144W+G77T+I165L+A292S+S278 R+Y69H+S141G-12aa, T291A+G292A+T70A+I160G+S204L+V244S+T134I+M326Q+Y144W+G77T+I165L+A292S+S278 R+Y69H+G140C -12aa, T291A+G292A+T70G+I160G+S204L+V244S+T134I+M326Q+Y144W+G77T+I165L+A292S+S278 R+Y69H +Y139S-12aa, T291A+G292A+T70G+I160G+S204L+V244S+T134I+M326Q+Y144W+G77T+I165L+A292S+S278 R+Y69H +G160T-12aa, T291A+G292A+T70G+I160G+S204L+V2445+T134I+M326Q+Y144W+G77T+I165L+A2925+5278 R+Y69H +G160T+S292A-12aa, T291A+G292A+T70G+I160G+S204L+V2445+T134I+M326Q+Y144W+G77T+I165L+A2925+5278 R+Y69H +G160T+A291T-12aa, T291A+G292A+T70G+I160G+S204L+V2445+T134I+M326Q+Y144W+G77T+I165L+A2925+5278 R+Y69H +G160T+I134V-12aa, T291A+G292A+T70G+I160G+S204L+V2445+T134I+M326Q+Y144W+G77T+I165L+A2925+5278 R+Y69H+ G160T+T106S-12aa, or T291A+G292A+T70G+I160G+S204L+V244S+T134I+M326Q+Y144W+G77T+I165L+A292S+S278 R+Y69H +G160T+Q171T-12aa.

4. A DNA molecule, wherein the DNA molecule encodes the transaminase mutant according to any one of claims 1 to 3.

5. A recombinant plasmid, wherein the recombinant plasmid contains the DNA molecule according to claim 4.

6. The recombinant plasmid according to claim 5, wherein the recombinant plasmid is pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18 or pUC-19.

7. A host cell, wherein the host cell contains the recombinant plasmid according to claim 5 or 6.

8. The host cell according to claim 7, wherein the host cell comprises a prokaryotic cell or a eukaryotic cell.

9. The host cell according to claim 8, wherein the prokaryotic cell is *Escherichia coli* BL21-DE3 cell or *Escherichia coli* Rosetta-DE3 cell; and the eukaryotic cell is yeast.

10. A method for producing a chiral amine, comprising a step of using a transaminase to catalyze a transamination reaction of a ketone compound and an amino donor, wherein the transaminase is the transaminase mutant according to any one of claim 1 to 3.

11. The method according to claim 10, wherein the ketone compound is 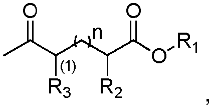 wherein, R₁ is H, an unsubstituted or optionally halogen-substituted C1-C10 alkyl, an unsubstituted or optionally halogen-substituted C5-C10 cycloalkyl, and an unsubstituted or optionally halogen-substituted C5-C10 aryl or C5-C10 heteroaryl, R₂ is H, a halogen, and a C1-C10 alkyl unsubstituted or optionally substituted with the halogen, NH₃ or at least one group in NH₃ protected with Boc and Cbz, and R₃ is an unsubstituted or optionally halogen-substituted C6-12 aryl, and an unsubstituted or optionally halogen-substituted C3-6 heterocyclyl.

12. The method according to claim 11, wherein the ketone compound is

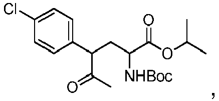

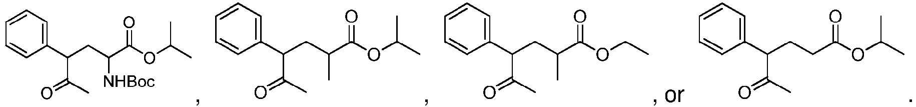

13. The method according to claim 10, wherein the amino donor is an isopropylamine or an alanine.

14. The method according to claim 13, wherein the amino donor is the isopropylamine.

15. The method according to claim 10, wherein in a reaction system in which the transaminase catalyzes the transamination reaction of the ketone compound and the amino donor, pH is 7-11.

16. The method according to claim 15, wherein in the reaction system in which the transaminase catalyzes the transamination reaction of the ketone compound and the amino donor, pH is 8-10.5.

17. The method according to claim 16, wherein in the reaction system in which the transaminase catalyzes the transamination reaction of the ketone compound and the amino donor, pH is 10.5.

18. The method according to claim 15, wherein in the reaction system in which the transaminase catalyzes the transamination reaction of the ketone compound and the amino donor, the temperature is 25-60°C.

19. The method according to claim 18, wherein in the reaction system in which the transaminase catalyzes the transamination reaction of the ketone compound and the amino donor, the temperature is 35-55°C.

20. The method according to claim 19, wherein in the reaction system in which the transaminase catalyzes the transamination reaction of the ketone compound and the amino donor, the temperature is 50°C.

21. The method according to claim 15, wherein a volume concentration of dimethyl sulfoxide in the reaction system the transaminase catalyzes the transamination reaction of the ketone compound and the amino donor is 0% to 50%.
